(19) Europäisches Patentamt
European Patent Office
Office européen des brevets

(11) EP 3 252 841 B1

(12) EUROPEAN PATENT SPECIFICATION

(45) Date of publication and mention of the grant of the patent:
**01.04.2026 Bulletin 2026/14**

(21) Application number: **16172006.5**

(22) Date of filing: **30.05.2016**

(51) International Patent Classification (IPC):
***H10K 50/12*** *(2023.01)* ***H10K 50/14*** *(2023.01)*
***H10K 71/30*** *(2023.01)* ***H10K 85/60*** *(2023.01)*
***H10K 85/30*** *(2023.01)*

(52) Cooperative Patent Classification (CPC):
**H10K 71/30; H10K 50/12; H10K 50/157; H10K 85/351; H10K 85/6572;** H10K 50/828

# (54) ORGANIC LIGHT EMITTING DIODE COMPRISING AN ORGANIC SEMICONDUCTOR LAYER

ORGANISCHE LICHTEMITTIERENDE DIODE MIT EINER ORGANISCHEN HALBLEITERSCHICHT

DIODE ÉLECTROLUMINESCENTE ORGANIQUE COMPRENANT UNE COUCHE SEMI-CONDUCTRICE ORGANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**06.12.2017 Bulletin 2017/49**

(73) Proprietor: **Novaled GmbH**
**01099 Dresden (DE)**

(72) Inventors:
- **Burkhardt, Martin**
  **01307 Dresden (DE)**
- **Huang, Qiang**
  **01108 Dresden (DE)**
- **Rothe, Carsten**
  **01326 Dresden (DE)**
- **Rosenow, Thomas**
  **01069 Dresden (DE)**

(74) Representative: **Bittner, Thomas L.**
**Boehmert & Boehmert**
**Anwaltspartnerschaft mbB**
**Patentanwälte Rechtsanwälte**
**Kurfürstendamm 185**
**10707 Berlin (DE)**

(56) References cited:
**EP-A1- 2 833 429** **EP-A1- 2 833 700**
**JP-A- 2008 177 459** **JP-A- 2008 243 932**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 252 841 B1

## Description

The present invention relates to an organic light emitting diode (OLED) comprising an organic semiconductor layer and a method of manufacturing the organic light emitting diode (OLED) comprising the organic semiconductor layer.

DESCRIPTION OF THE RELATED ART

Organic light emitting diodes (OLEDs), which are self-emitting devices, have a wide viewing angle, excellent contrast, quick response, high brightness, excellent driving voltage characteristics, and color reproduction. A typical OLED includes an anode electrode, a hole injection layer (HIL), a hole transport layer (HTL), an emission layer (EML), an electron transport layer (ETL), and a cathode electrode, which are sequentially stacked on a substrate. In this regard, the HIL, the HTL, the EML, and the ETL are thin films formed from organic compounds.

When a voltage is applied to the anode electrode and the cathode electrode, holes injected from the anode electrode move to the EML, via the HIL and HTL, and electrons injected from the cathode electrode move to the EML, via the ETL. The holes and electrons recombine in the EML to generate excitons.

Semiconductor layers comprised in organic light emitting diodes of the art may be formed by depositing an organic matrix material together with a metal dopant, such as Cs or Li. However, such OLEDs of the prior art may suffer from poor performance. Further, when preparing these OLEDs, poor control over evaporation rate of the metal dopant is a significant problem. In particular, the doping concentration of Li is very low and therefore difficult to control. Furthermore, safe handling of the metal dopant is highly desirable, both when loading the vacuum thermal evaproration (VTE) source and when opening up the evaporation chamber for maintenances.

EP 2 833 429 A1 discloses an organic electroluminescence device including: an anode; one or more organic thin film layers including an emitting layer; a donor-containing layer; an acceptor-containing layer; and a light-transmissive cathode in this order.

JP 2008 243932 A discloses an organic electroluminescent element, an anode, a luminous function layer and a light-transmitting cathode are laminated in this order.

JP 2008 177459 A discloses an organic electric field light emitting element, the light emitting function layer includes an optically transparent negative electrode, an electron injection layer and a light emitting layer which are laminated in this order, wherein the electron injection layer is produced by doping a compound of an alkali metal, an element belonging to the group II or a rare earth element in an organic material.

EP 2 833 700 A1 discloses an organic electroluminescence device including: an anode; a cathode; two or more emitting units that are disposed between the anode and the cathode, each unit having an emitting layer; and a charge-generating layer that is disposed between the emitting units, wherein the charge-generating layer includes an N layer nearer to the anode and a P layer nearer to the cathode.

SUMMARY

It is therefore the object of the present invention to provide an organic light emitting diode comprising metal doped organic semiconductor layers overcoming drawbacks of the prior art, in particular having improved operating voltage, external quantum efficiency and / or voltage rise over time. Furthermore, it is the object of the present invention to provide metal dopants which are suitable for manufacturing OLEDs having reduced air-sensitivity and good control over the evaporation rate thereof.

The above problems are solved in accordance with the subject matter of the independent claims. Preferred embodiments result from the subclaims.

This object is achieved by Organic light emitting diode (100) comprising an anode electrode, a cathode electrode, at least one emission layer and at least one organic semiconductor layer, wherein the at least one emission layer and the at least one organic semiconductor layer are arranged between the anode electrode and the cathode electrode and the at least one organic semiconductor layer comprises a substantially metallic zero valent rare earth metal dopant selected from Sm, Eu, and Yb and

a first matrix compound, the first matrix compound comprising two phenanthrolinyl groups, characterized in that

the organic light emitting diode comprises a first emission layer and a second emission layer,

wherein the organic semiconductor layer is arranged be-tween the first emission layer and the second emission layer;

the organic light emitting diode further comprises a p-type charge generation layer, wherein the organic semiconductor layer is arranged between the first emission layer and the p-type charge generation layer; and

the organic semiconductor layer is in direct contact with the p-type charge generation layer..

Preferably, the first matrix compound is a substantially organic compound. More preferred the first matrix compound has a molar mass of 450 to 1100 gramm per mole.
The term "substantially organic" shall be understood to encompass compounds which contain the elements C, H, N, O, S, B, P, or Si and are free of metals.
More preferred, the phenanthrolinyl groups are comprised in the first matrix compound by covalent bonding via the three-valent carbon atom adjacent to one of the two nitrogen atoms comprised in the phenanthrolinyl moiety.
Preferably, the first matrix compound is a compound of Formula 1

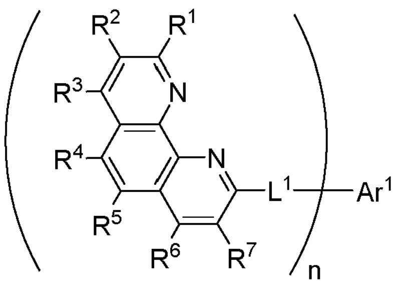

Formula 1

wherein $R^1$ to $R^7$ are each independently selected from the group consisting of hydrogen, substituted or unsubstituted $C_6$ to $C_{18}$ aryl group, substituted or unsubstituted pyridyl group, substituted or unsubstituted quinolyl group, substituted or unsubstituted $C_1$ to $C_{16}$ alkyl group, substituted or unsubstituted $C_1$ to $C_{16}$ alkoxy group, hydroxyl group or carboxyl group, and/or wherein adjacent groups of the respective $R^1$ to $R^7$ may be bonded to each other to form a ring;

$L^1$ is a single bond or selected from a group consisting of a $C_6$ to $C_{30}$ arylene group, a $C_5$ to $C_{30}$ heteroarylene group, a $C_1$ to $C_8$ alkylene group or a $C_1$ to $C_8$ alkoxyalkylene group;

$Ar^1$ is a substituted or unsubstituted $C_6$ to $C_{18}$ aryl group or a pyridyl group; and

n is 2.

The term "alkyl" as used herein shall encompass linear as well as branched and cyclic alkyl. For example, $C_3$-alkyl may be selected from n-propyl and iso-propyl. Likewise, $C_4$-alkyl encompasses n-butyl, sec-butyl and t-butyl. Likewise, $C_6$-alkyl encompasses n-hexyl and cyclohexyl.
The subscribed number n in $C_n$ relates to the total number of carbon atoms in the respective alkyl, aryl, heteroaryl or alkoxy group.
The term "aryl" as used herein shall encompass phenyl ($C_6$-aryl), fused aromatics, such as naphthalene, anthracene, phenanthracene, tetracene etc. Further encompassed are biphenyl and oligo- or polyphenyls, such as terphenyl etc. Further encompassed shall be any further aromatic hydrocarbon substituents, such as fluorenyl etc. Arylene refers to groups to which two further moieties are attached.
The term "heteroaryl" as used herewith refers to aryl groups in which at least one carbon atom is substituted by a heteroatom, preferably selected from N, O, S, B or Si. Heteroarylene refers to groups to which two further moieties are attached.
Likewise, the term "alkoxy" as used herein refers to alkoxy groups (-O-alkyl) wherein the alkyl is defined as above.
The subscribed number n in $C_n$-heteroaryl merely refers to the number of carbon atoms excluding the number of heteroatoms. In this context, it is clear that a $C_5$ heteroarylene group is an aromatic compound comprising five carbon atoms, such as pyridyl.
According to the invention, if the respective groups are $R^1$ to $R^7$, $L^1$ and $Ar^1$ are substituted, the groups may preferably be substituted with at least one $C_1$ to $C_{12}$ alkyl group or $C_1$ to $C_{12}$ alkoxy group, more preferably $C_1$ to $C_4$ alkyl group or $C_1$ to $C_4$ alkoxy group. By appropriately choosing the respective substituents, in particular the length of the hydrocarbon chains, the physical properties of the compounds, for example solubility of the same in organic solvents or evaporation rate, can be adjusted.
In a further preferred embodiment, $L^1$ is a single bond.
Preferably, $Ar^1$ is phenylene.
More preferred, $R^1$ to $R^7$ are independently selected from the group consisting of hydrogen, $C_1$ to $C_4$ alkyl group,

$C_1$ to $C_4$ alkoxy group, $C_6$ to $C_{12}$ aryl group and $C_5$ to $C_{12}$ heteroaryl group, preferably from hydrogen, $C_1$ to $C_4$ alkyl group and phenyl.

Preferably, the first matrix compound is selected from the group consisting of

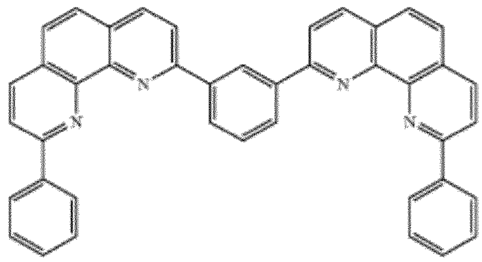
MX1,

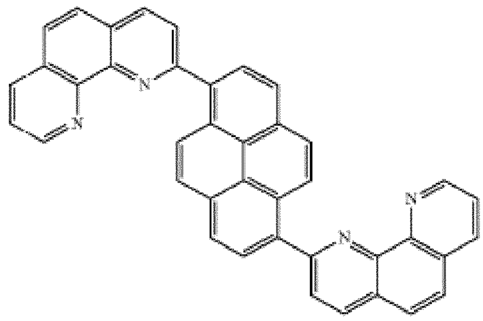
MX2,

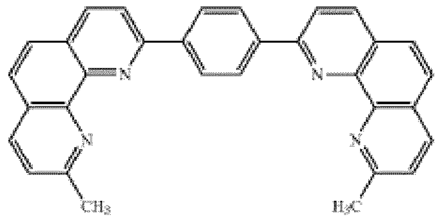
MX3,

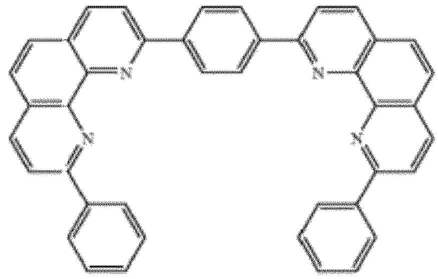
MX4,

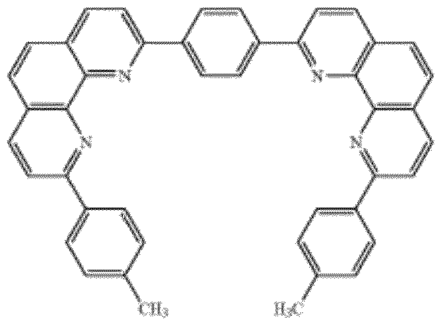
MX5,

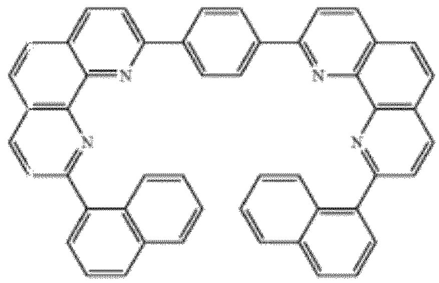
MX6,

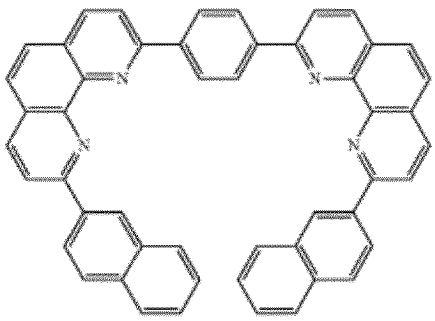
MX7,

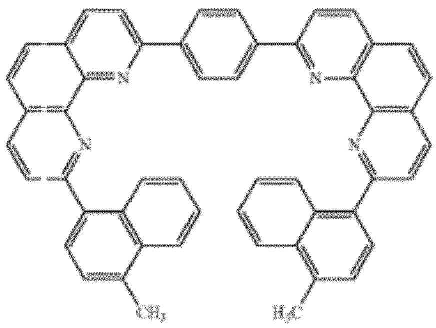
MX8,

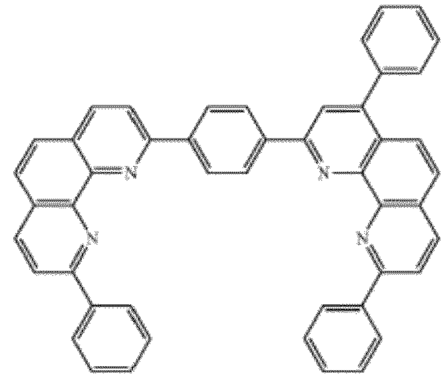

MX9,

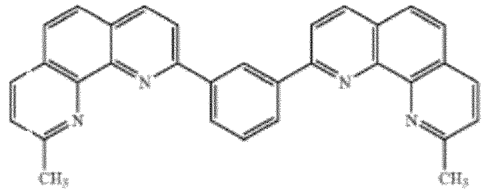

MX10,

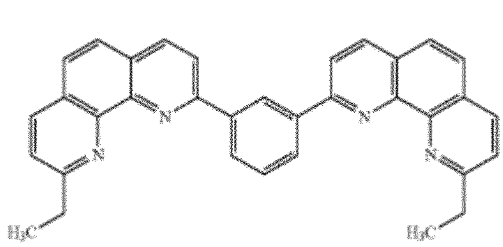

MX11,

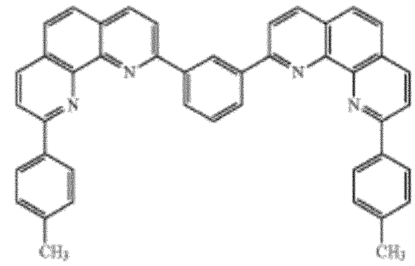

MX12,

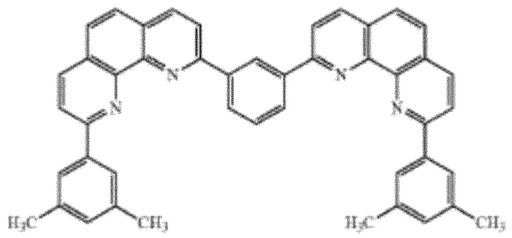

MX13,

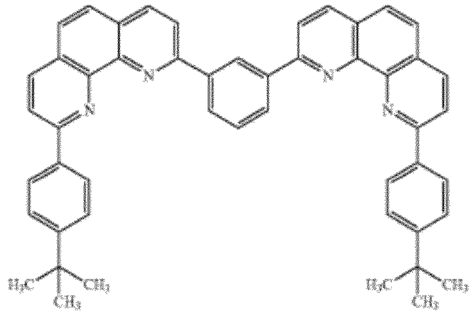

MX14,

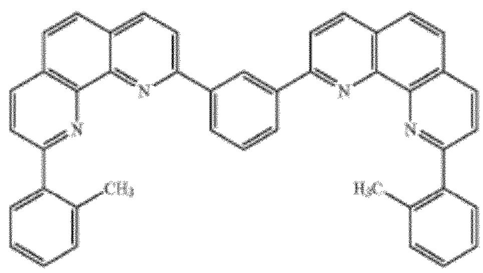

MX15,

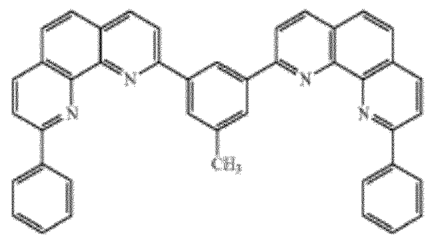

MX16,

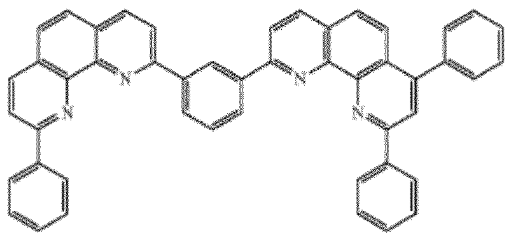

MX17,

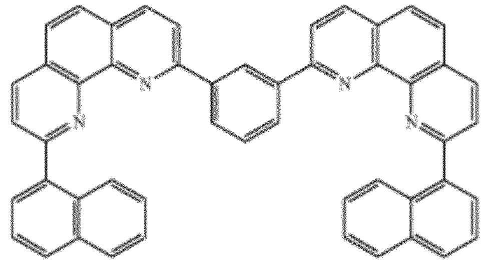

MX18,

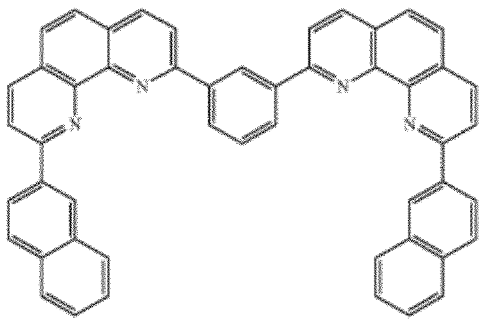
MX19,

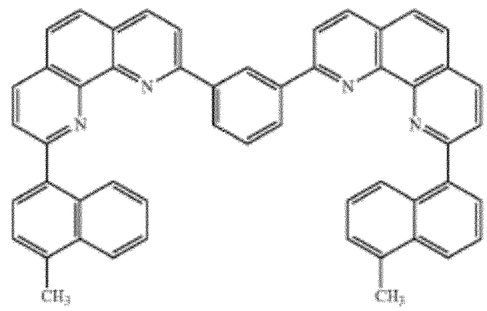
MX20,

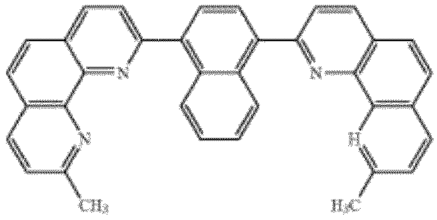
MX21,

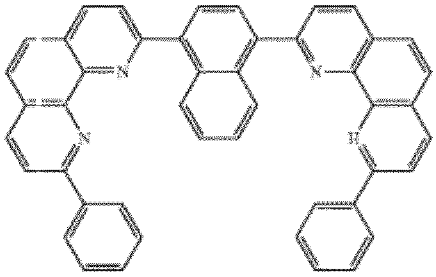
MX22,

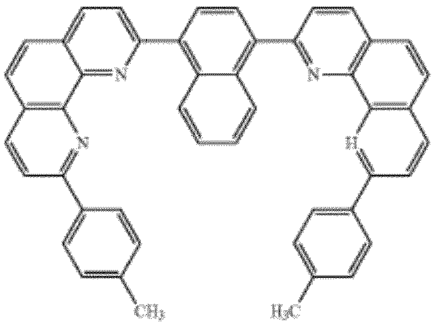
MX23,

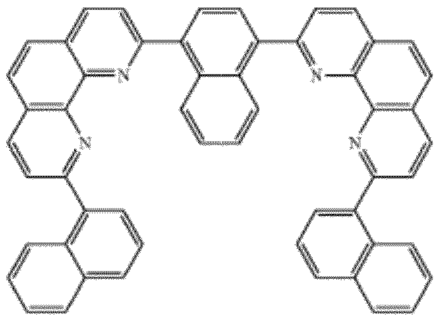
MX24,

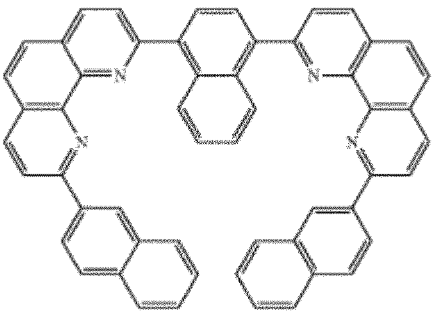
MX25,

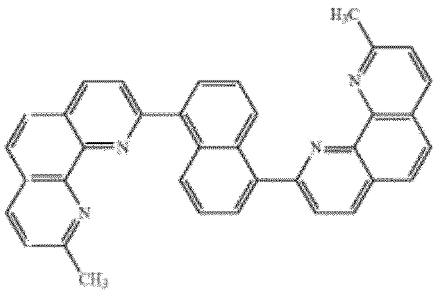
MX26,

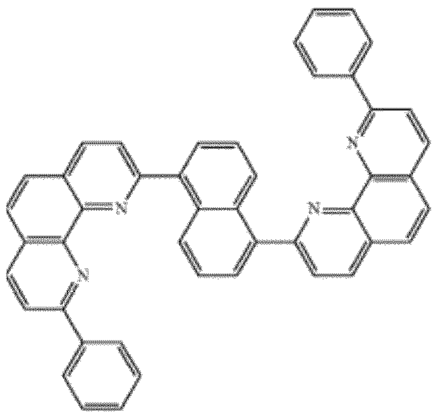

MX27,

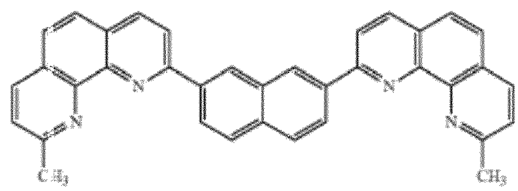

MX28,

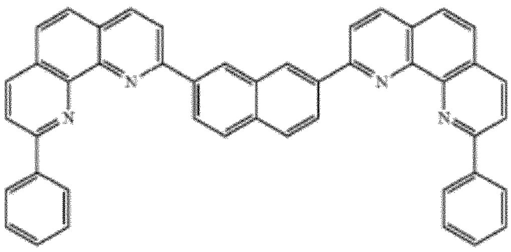

MX29,

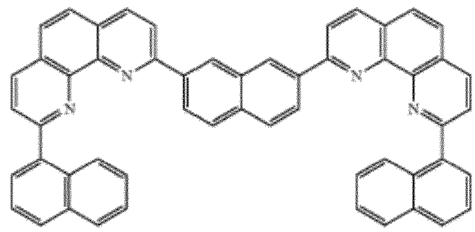

MX30,

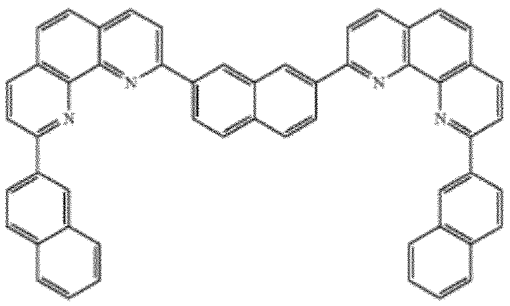

MX31,

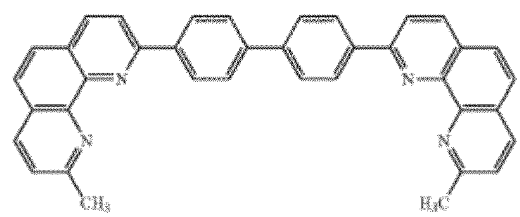

MX32,

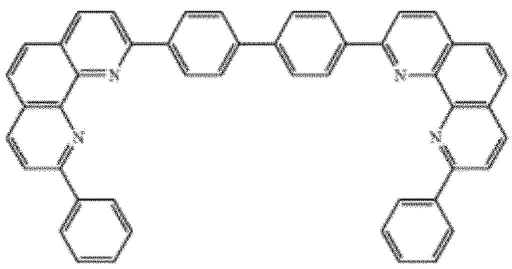

MX33,

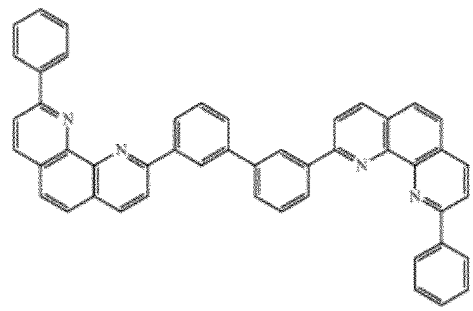

MX34,

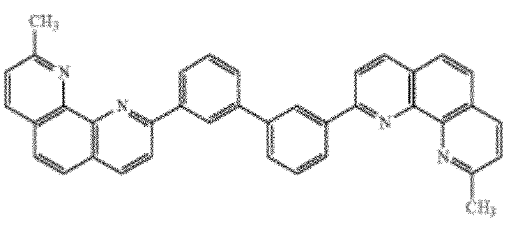

MX35,

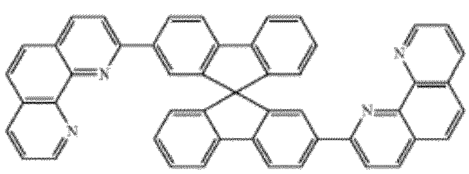

MX36,

In this regard, it is most preferred that the first matrix compound is

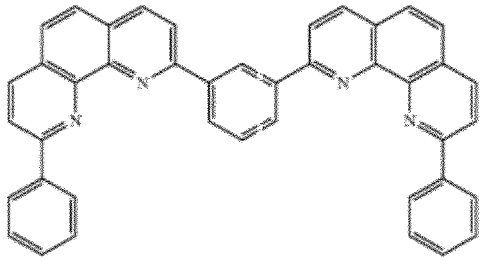

MX1.

It is most preferred that the substantially metallic rare earth metal dopant is Yb.

The term "substantially metallic" shall be understood as encompassing a metal which is at least partially in a substantially elemental form. The term "substantially elemental" is to be understood as a form that is, in terms of electronic states and energies and in terms of chemical bonds of comprised metals atoms closer to the form of an elemental metal, or a free metal atom or to the form of a cluster of metal atoms, then to the form of a metal salt, of an organometallic metal compound or another compound comprising a covalent bond between metal and non-metal, or to the form of a coordination compound of a metal.

One benefit offered by rare earth metal dopants is the higher doping concentration when measured in weight percent compared to alkali metals, in particular compared to lithium. Thereby, good control over the evaporation rate may be achieved and improved reproducibility may be obtained in manufacturing processes. Additionally, rare earth metals are less air- and moisture-sensitive than alkali metals and alkaline earth metals and therefore are safer to use in mass production. In a further aspect, rare earth metal dopants are less prone to diffusion than alkali metals and alkaline earth metals. Therefore, stability over time may be improved, for example rise of operating voltage over time.

According to the invention, the organic light emitting diode comprises a first emission layer and a second emission layer, wherein the organic semiconductor layer is arranged between the first emission layer and the second emission layer.

In another embodiment, the organic light emitting diode comprises a first organic semiconductor layer and a second organic semiconductor layer, wherein the first organic semiconductor layer is arranged between the first emission layer and the second emission layer and the second organic semiconductor layer is arranged between the cathode electrode and the emission layer closest to the cathode electrode. Thereby, excellent performance may be achieved while using only the same compounds in the n-type charge generation layer and the electron transport and / or electron injection layer.

According to the invention, the organic light emitting diode further comprises a p-type charge generation layer, wherein the organic semiconductor layer is arranged between the first emission layer and the p-type charge generation layer. Thereby, generation and transport of electrons between the first and second emission layer may be improved.

The organic semiconductor layer is in direct contact with the p-type charge generation layer.

Preferably, the organic semiconductor layer is not the cathode electrode. The cathode electrode is substantially metallic. Preferably, the cathode electrode is free of organic compounds.

In another embodiment, the at least one organic semiconductor layer is not in direct contact with the at least one emission layer. Thereby, quenching of light emission through the substantially metallic rare earth metal dopant may be reduced.

Preferably, the organic semiconductor layer is essentially non-emissive.

In the context of the present specification the term "essentially non-emissive" means that the contribution from the organic semi-conductor layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

In another embodiment, the organic light emitting diode further comprises an electron transport layer which is arranged between the at least one emission layer and the at least one organic semi-conductor layer.

In another embodiment, the p-type charge generation layer is arranged between the organic semi-conductor layer and the cathode electrode.

In another embodiment, the cathode electrode is transparent to visible light emission.

In this regard, the term "transparent" refers to the physical property of allowing at least 50% of visible light emission to pass through the material, preferably at least 80%, more preferably at least 90%.

In another embodiment, the anode electrode and cathode electrode may be transparent to visible light emission.

In another embodiment, the cathode electrode comprises a first cathode electrode layer and a second cathode electrode layer.

In this regard, the first cathode electrode layer and/or the second cathode electrode layer are obtainable by depositing the same using a sputtering process. Preferably the second electrode is formed by using a sputtering process.

Furthermore, the object is achieved by a method of manufacturing an inventive organic light emitting diode, comprising the steps of sequentially forming an anode electrode, at least one emission layer, at least one organic semiconductor layer, and a cathode electrode on a substrate, and forming the at least one organic semiconductor layer by co-depositing the substantially metallic zero valent rare earth metal dopant selected from Sm, Eu, and Yb together with the first matrix compound comprising two phenanthrolinyl groups.

According to various embodiments of the organic light emitting diode of the present invention the thicknesses of the organic semiconductor layer can be in the range of about ≥ 5 nm to about ≤ 500 nm, preferably of about ≥ 10 nm to about ≤ 200 nm.

If the cathode electrode is deposited through a sputtering process, the thickness of the organic semiconductor layer is preferably in the range of ≥ 100 nm to about ≤ 500 nm.

The thickness of the organic semiconductor layer is preferably in the range of about ≥ 5 nm to about ≤ 100 nm, more preferred in the range of about ≥ 5 nm to about ≤ 40 nm.

In the present invention, the following defined terms, these definitions shall be applied, unless a different definition is given in the claims or elsewhere in this specification.

In the context of the present specification the term "different" or "differs" in connection with the matrix material means that the matrix material differs in their structural formula.

In the context of the present specification the term "different" or "differs" in connection with the lithium compound means that the lithium compound differs in their structural formula.

The term "free of", "does not contain", "does not comprise" does not exclude impurities which may be present in the compounds prior to deposition. Impurities have no technical effect with respect to the object achieved by the present invention.

Vacuum thermal evaporation, also named VTE, describes the process of heating a compound in a VTE source and evaporating said compound from the VTE source under reduced pressure.

The external quantum efficiency, also named EQE, is measured in percent (%).

The lifetime, also named LT, between starting brightness and 97 % of the original brightness is measured in hours (h).

The operating voltage, also named V, is measured in Volt (V) at 10 milliAmpere per square centimeter ($mA/cm^2$).

The voltage rise over time, also named V rise, is measured in Volt (V) at 30 milliAmpere per square centimeter ($mA/cm^2$) and a temperature of 85 °C.

The color space is described by coordinates CIE-x and CIE-y (International Commission on Illumination 1931). For blue emission the CIE-y is of particular importance. A smaller CIE-y denotes a deeper blue color.

The highest occupied molecular orbital, also named HOMO, and lowest unoccupied molecular orbital, also named LUMO, are measured in electron volt (eV).

The terms "OLED" and "organic electroluminescent device", "organic light-emitting diode" and "organic light emitting diode" are simultaneously used and have the same meaning.

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is understood that the total weight percent amount of all components, substances and agents of the respective organic semi-conductor layer are selected such that it does not exceed 100 wt.-%.

As used herein, "mol percent", "mol.-%", "percent by mol", "% by mol", and variations thereof refer to a composition, component, substance or agent as the molar mass of that component, substance or agent of the respective electron transport layer divided by the total molar mass of the respective electron transport layer thereof and multiplied by 100. It is understood that the total mol percent amount of all components, substances and agents of the respective organic semiconductor layer are selected such that it does not exceed 100 mol.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

Herein, when a first element is referred to as being formed or disposed "on" a second element, the first element

can be disposed directly on the second element or one or more other elements may be disposed there between. When a first element is referred to as being formed or disposed "directly on" a second element, no other elements are disposed there between.

The term "contacting sandwiched" refers to an arrangement of three layers whereby the layer in the middle is in direct contact with the two adjacent layers.

The anode electrode and cathode electrode may be described as anode electrode / cathode electrode or anode electrode / cathode electrode or anode electrode layer / cathode electrode layer.

The organic light emitting diode according to the invention may comprise the following constituents. In this regard, the respective constituents may be as follows.

Substrate

The substrate may be any substrate that is commonly used in manufacturing of organic light-emitting diodes. If light is emitted through the substrate, the substrate may be a transparent material, for example a glass substrate or a transparent plastic substrate, having excellent mechanical strength, thermal stability, transparency, surface smoothness, ease of handling, and waterproofness. If light is emitted through the top surface, the substrate may be a transparent or non-transparent material, for example a glass substrate, a plastic substrate, a metal substrate or a silicon substrate.

Anode electrode

The anode electrode may be formed by depositing or sputtering a compound that is used to form the anode electrode. The compound used to form the anode electrode may be a high work-function compound, so as to facilitate hole injection. The anode material may also be selected from a low work function material (i.e. Aluminum).The anode electrode may be a transparent or reflective electrode. Transparent conductive compounds, such as indium tin oxide (ITO), indium zinc oxide (IZO), tin-dioxide ($SnO_2$), and zinc oxide (ZnO), may be used to form the anode electrode 120.

The anode electrode 120 may also be formed using magnesium (Mg), aluminum (Al), aluminum-lithium (Al-Li), calcium (Ca), magnesium-indium (Mg-In), magnesium-silver (Mg-Ag), silver (Ag), gold (Au), or the like.

Cathode electrode

In a further preferred embodiment, the cathode electrode comprises at least one substantially metallic cathode layer comprising a first zero-valent metal selected from the group consisting of alkali metal, alkaline earth metal, rare earth metal, group 3 transition metal and mixtures thereof.

The term "substantially metallic" shall be understood as encompassing a metal which is at least partially in a substantially elemental form. The term substantially elemental is to be understood as a form that is, in terms of electronic states and energies and in terms of chemical bonds of comprised metals atoms closer to the form of an elemental metal, or a free metal atom or to the form of a cluster of metal atoms, then to the form of a metal salt, of an organometallic metal compound or another compound comprising a covalent bond between metal and non-metal, or to the form of a coordination compound of a metal.

It is to be understood that metal alloys represent beside neat elemental metals, atomized metals, metal molecules and metal clusters, any other example of substantially elemental form of metals. These exemplary representatives of substantially metallic forms are the preferred substantially metallic cathode layer constituents.

Particularly low operating voltage and high manufacturing yield may be obtained when the first zero-valent metal is selected from this group.

According to another aspect there is provided an organic light emitting diode wherein the substantially metallic cathode layer is free of a metal halide and/or free of a metal organic complex.

According to a preferred embodiment, the substantially metallic cathode electrode layer comprises or consists of the first zero-valent metal. In particularly preferred embodiments, the substantially metallic cathode layer further comprises a second zero-valent metal, wherein the second zero-valent metal is selected from a main group metal or a transition metal; and wherein the second zero-valent metal is different from the first zero-valent metal.

In this regard, it is further preferred that the second zero-valent metal is selected from the group consisting of Li, Na, K, Cs, Mg, Ca, Sr, Ba, Sc, Y, Ti, V, Cr, Mn, Fe, Co, Ni, Cu, Zn, Ag, Au, Al, Ga, In, Sn, Te, Bi, Pb and mixtures thereof, preferably the second zero-valent metal is selected from the group consisting of Ag, Au, Zn, Te, Yb, Ga, Bi, Ba, Ca, Al and mixtures thereof; and most preferred the second zero-valent metal is selected from the group consisting of Ag, Zn, Te, Yb, Ga, Bi and mixtures thereof.

The second zero-valent metal may improve reliability of the deposition process and mechanical stability of the deposited layer and thereby improve manufacturing yield when selected from this list. Additionally, the second zero-valent metal may improve reflectivity of the first cathode electrode layer.

According to another embodiment the substantially metallic cathode layer can comprises at least about ≥ 15 vol.-% to about ≤ 99 vol.-% of the first zero-valent metal and less than about ≥ 85 vol.-% to about ≤ 1 vol.-% of the second zero-valent metal; preferably ≥ 15 vol.-% to about ≤ 95 vol.-% of the first zero-valent metal and less than about ≥ 85 vol.-% to about ≤ 5 vol.-% of the second zero-valent metal; more preferred ≥ 20 vol.-% to about ≤ 90 vol.-% of the first zero-valent metal and less than about ≥ 80 vol.-% to about ≤ 10 vol.-% of the second zero-valent metal; also preferred ≥ 15 vol.-% to about ≤ 80 vol.-% of the first zero-valent metal and less than about ≥ 85 vol.-% to about ≤ 20 vol.-% of the second zero-valent metal.

Particularly preferred the substantially metallic cathode layer comprises at least about ≥ 20 vol.-% to about ≤ 85 vol.-% of the first zero-valent metal, selected from Mg and less than about ≥ 80 vol.-% to about ≤ 15 vol.-% of the second zero-valent metal selected from Ag.

The first zero-valent metal may enable efficient electron injection from the cathode. The second zero-valent metal may stabilize the cathode layer and / or increase yield of the cathode deposition step and / or increase transparency or reflectivity of the cathode.

In a further embodiment, the substantially metallic cathode layer comprised in the cathode electrode is a first cathode layer and the cathode electrode further comprises a second cathode layer, wherein the first cathode layer is arranged closer to the organic semiconductor layer and the second cathode layer is arranged further away from the organic semiconductor layer and wherein the second cathode layer comprising at least one third metal in form of a zero-valent metal, an alloy, an oxide or a mixture thereof, wherein the third metal is selected from a main group metal, transition metal, rare earth metal or mixtures thereof, preferably the third metal is selected from zero-valent Ag, Al, Cu, Au, MgAg alloy, indium tin oxide, indium zinc oxide, ytterbium oxide, indium gallium zinc oxide and more preferred the third metal is selected from Ag, Al, or MgAg alloy; and most preferred the third metal is selected from zero-valent Ag or Al.

The second cathode electrode layer may protect the first cathode electrode layer from the environment. Additionally it may enhance outcoupling of light emission in devices when light is emitted through the cathode electrode.

The thickness of the first cathode electrode layer may be in the range of about 0.2 nm to 100 nm, preferably 1 to 50 nm. If no second cathode electrode layer is present, the thickness of the first cathode electrode layer may be in the range of 1 to 25 nm. If a second cathode electrode layer is present, the thickness of the first cathode electrode layer may be in the range of 0.2 to 5 nm.

The thickness of the second cathode electrode layer may be in the range of 0.5 to 500 nm, preferably 10 to 200 nm, even more preferred 50 to 150 nm.

When the thickness of the cathode electrode is in the range of 5 nm to 50 nm, the cathode electrode may be transparent even if a metal or metal alloy is used.

In a further embodiment, the cathode electrode comprises transparent conductive oxide (TCO), metal sulfide and / or Ag, preferably indium tin oxide (ITO), indium zinc oxide (IZO), zinc sulfide or Ag. Most preferred are ITO and Ag. If the cathode electrode comprises these compounds it may be transparent to visible light emission.

The thickness of the transparent cathode electrode may be in the range of 5 to 500 nm. If the transparent cathode electrode consists of transparent conductive oxide (TCO) or metal sulfide, the thickness of the transparent cathode electrode may be selected in the range of 30 to 500 nm, preferably 50 to 400 nm, even more preferred 70 to 300 nm. If the transparent cathode electrode consists of Ag, the thickness of the transparent cathode electrode may be selected in the range of 5 to 50 nm, preferably 5 to 20 nm.

## Hole injection layer

The hole injection layer (HIL) 130 may be formed on the anode electrode 120 by vacuum deposition, spin coating, printing, casting, slot-die coating, Langmuir-Blodgett (LB) deposition, or the like. When the HIL 130 is formed using vacuum deposition, the deposition conditions may vary according to the compound that is used to form the HIL 130, and the desired structure and thermal properties of the HIL 130. In general, however, conditions for vacuum deposition may include a deposition temperature of 100° C to 500° C, a pressure of $10^{-8}$ to $10^{-3}$ Torr (1 Torr equals 133.322 Pa), and a deposition rate of 0.1 to 10 nm/sec.

When the HIL 130 is formed using spin coating, printing, coating conditions may vary according to a compound that is used to form the HIL 130, and the desired structure and thermal properties of the HIL 130. For example, the coating conditions may include a coating speed of about 2000 rpm to about 5000 rpm, and a thermal treatment temperature of about 80° C to about 200° C. Thermal treatment removes a solvent after the coating is performed.

The HIL 130 may be formed of any compound that is commonly used to form an HIL. Examples of compounds that may be used to form the HIL 130 include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL 130 may be a pure layer of p-dopant or may be selected from a hole-transporting matrix compound doped

with a p-dopant. Typical examples of known redox doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile (PD1). α-NPD doped with 2,2',2"-(cyclopropane-1,2,3-triylidene)tris(2-(p-cyanotetrafluorophenyl)acetonitrile) (PD2). Dopant concentrations can be selected from 1 to 20 wt.-%, more preferably from 3 wt.-% to 10 wt.-%.

The thickness of the HIL 130 may be in the range of about 1 nm to about 100 nm, and for example, about 1 nm to about 25 nm. When the thickness of the HIL 130 is within this range, the HIL 130 may have excellent hole injecting characteristics, without a substantial increase in driving voltage.

Hole transport layer

The hole transport layer (HTL) 140 may be formed on the HIL 130 by vacuum deposition, spin coating, slot-die coating, printing, casting, Langmuir-Blodgett (LB) deposition, or the like. When the HTL 140 is formed by vacuum deposition or spin coating, the conditions for deposition and coating may be similar to those for the formation of the HIL 130. However, the conditions for the vacuum or solution deposition may vary, according to the compound that is used to form the HTL 140.

The HTL 140 may be formed of any compound that is commonly used to form a HTL. Compound that can be suitably used is disclosed for example in Yasuhiko Shirota and Hiroshi Kageyama, Chem. Rev. 2007, 107, 953- 1010. Examples of the compound that may be used to form the HTL 140 are: a carbazole derivative, such as N-phenylcarbazole or poly-vinylcarbazole; an amine derivative having an aromatic condensation ring, such as N,N'-bis(3-methylphenyl)-N,N'-diphenyl-[1,1-biphenyl]-4,4'-diamine (TPD), or N,N'-di(naphthalen-1-yl)-N,N'-diphenyl benzydine (alpha -NPD); and a triphenylamine-based compound, such as 4,4',4"-tris(N-carbazolyl)triphenylamine (TCTA). Among these compounds, TCTA can transport holes and inhibit excitons from being diffused into the EML.

The thickness of the HTL 140 may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL 140 may be 170 nm to 200 nm.

When the thickness of the HTL 140 is within this range, the HTL 140 may have excellent hole transporting characteristics, without a substantial increase in driving voltage.

Electron blocking layer

The function of the electron blocking layer (EBL) 150 is to prevent electrons from being transferred from the emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer is selected between 2 and 20 nm.

The electron blocking layer may comprise a compound of formula Z below

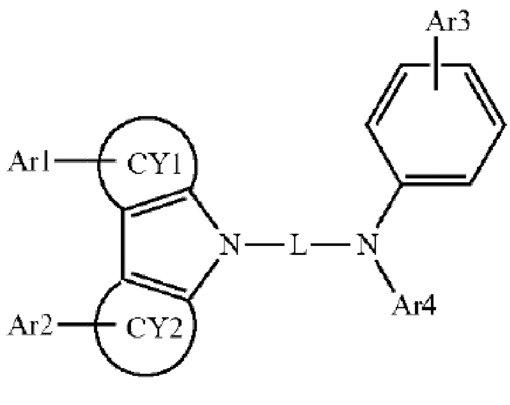

(Z).

**In** Formula Z,

CY1 and CY2 are the same as or different from each other, and each independently represent a benzene cycle or a naphthalene cycle,

Ar1 to Ar3 are the same as or different from each other, and each independently selected from the group consisting of hydrogen; a substituted or unsubstituted aryl group having 6 to 30 carbon atoms; and a substituted or unsubstituted heteroaryl group having 5 to 30 carbon atoms,

Ar4 is selected from the group consisting of a substituted or unsubstituted phenyl group, a substituted or unsubstituted biphenyl group, a substituted or unsubstituted terphenyl group, a substituted or unsubstituted triphenylene group, and a substituted or unsubstituted heteroaryl group having 5 to 30 carbon atoms,

L is a substituted or unsubstituted arylene group having 6 to 30 carbon atoms.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable triplet control layer, in particular the triarylamine compounds, are described in EP 2 722 908 A1.

Emission layer (EML)

The EML 150 may be formed on the HTL by vacuum deposition, spin coating, slot-die coating, printing, casting, LB, or the like. When the EML is formed using vacuum deposition or spin coating, the conditions for deposition and coating may be similar to those for the formation of the HIL. However, the conditions for deposition and coating may vary, according to the compound that is used to form the EML.

The emission layer (EML) may be formed of a combination of a host and a dopant. Example of the host are Alq3, 4,4'-N,N'- dicarbazole-biphenyl (CBP), poly(n-vinylcarbazole) (PVK), 9,10-di(naphthalene-2-yl)anthracene (ADN), 4,4',4"-Tris(carbazol-9-yl)-triphenylamine

(TCTA), 1,3,5-tris(N-phenylbenzimidazole-2-yl)benzene (TPBI), 3-tert-butyl-9,10-di-2-naphthylanthracenee (TBADN), distyrylarylene (DSA), Bis(2-(2-hydroxyphenyl)benzothiazolate)zinc (Zn(BTZ) 2), E3 below, AND, Compound 1 below, and Compound 2 below.

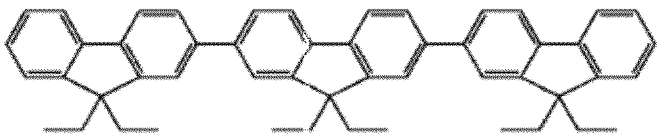

**E3**

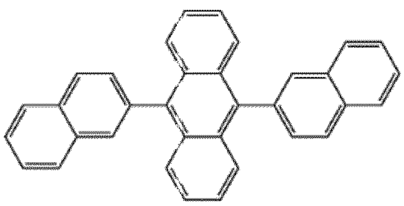

AND

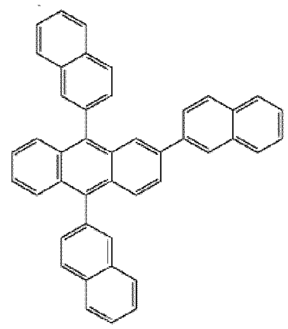

**Compound 1**

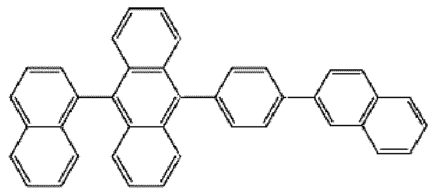

**Compound 2**

The dopant may be a phosphorescent or fluorescent emitter. Phosphorescent emitters and emitters which emit light via a thermally activated delayed fluorescence (TADF) mechanism are preferred due to their higher efficiency. The emitter may be a small molecule or a polymer.

Examples of a red dopant are PtOEP, $Ir(piq)_3$, and $Btp_2Ir(acac)$, but are not limited thereto. These compounds are phosphorescent emitters, however, fluorescent red dopants could also be used.

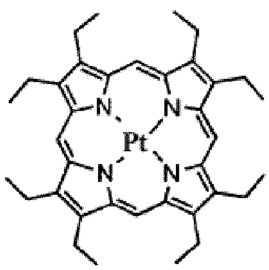

**PtOEP**

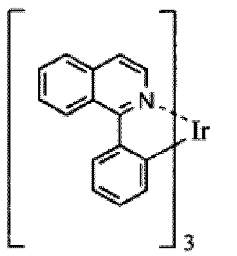

**$Ir(piq)_3$**

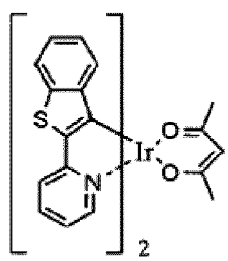

**$Btp_2Ir(acac)$**

Examples of a phosphorescent green dopant are $Ir(ppy)_3$ (ppy = phenylpyridine), $Ir(ppy)_2(acac)$, $Ir(mpyp)_3$ are shown below. Compound 3 is an example of a fluorescent green emitter and the structure is shown below.

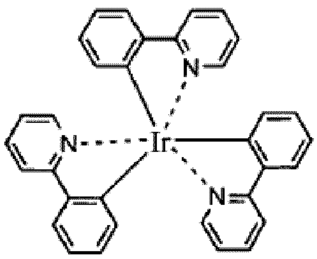
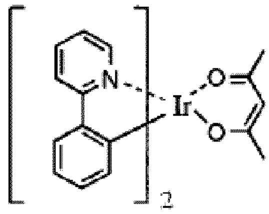
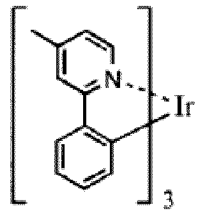

Ir(ppy)$_3$ Ir(ppy)$_2$(acac) Ir(mpyp)$_3$

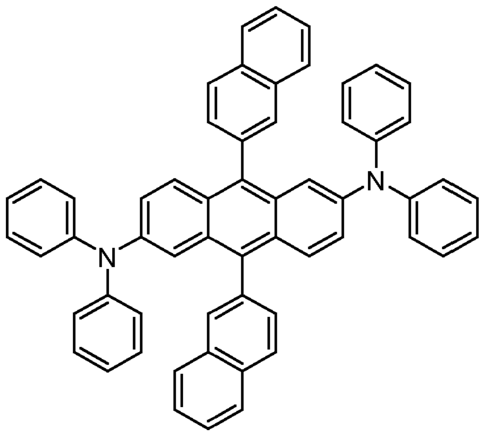

**Compound 3**

Examples of a phosphorescent blue dopant are $F_2$Irpic, ($F_2$ppy)$_2$Ir(tmd) and Ir(dfppz)$_3$, terfluorene, the structures are shown below. 4.4'-bis(4-diphenyl amiostyryl)biphenyl (DPAVBi), 2,5,8,11-tetra-tert-butyl perylene (TBPe), and Compound 4 below are examples of fluorescent blue dopants.

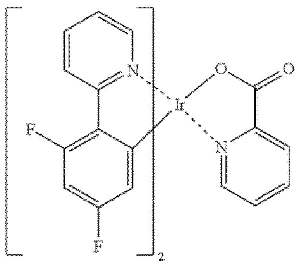
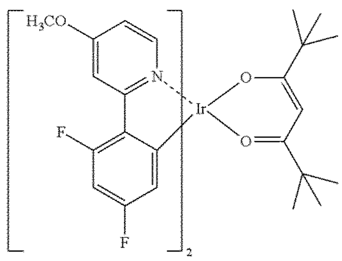
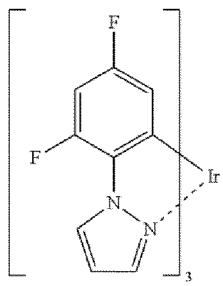

**$F_2$Irpic** **($F_2$ppy) $_2$Ir(tmd)** **Ir(dfppz) 3**

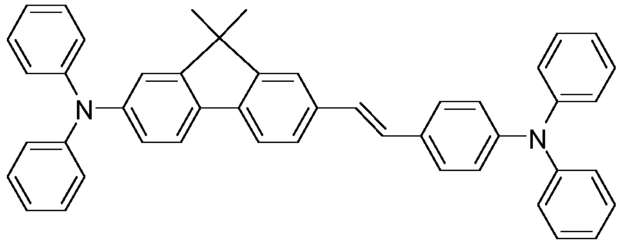

**Compound 4**

The amount of the dopant may be in the range of about 0.01 to about 50 parts by weight, based on 100 parts by weight of the host. Alternatively, the emission layer may consist of a light-emitting polymer. The EML may have a thickness of about 10 nm to about 100 nm, for example, about 20 nm to about 60 nm. When the thickness of the EML is within this range, the EML may have excellent light emission, without a substantial increase in driving voltage.

Hole blocking layer (HBL)

When the EML comprises a phosphorescent dopant, a hole blocking layer (HBL) may be formed on the EML, by using vacuum deposition, spin coating, slot-die coating, printing, casting, LB deposition, or the like, in order to prevent the diffusion of triplet excitons or holes into the ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include an oxadiazole derivative, a triazole derivative, and a phenanthroline derivative.

The HBL may have a thickness of about 5 nm to about 100 nm, for example, about 10 nm to about 30 nm. When the thickness of the HBL is within this range, the HBL may have excellent hole-blocking properties, without a substantial increase in driving voltage.

Electron transport layer (ETL)

The OLED according to the present invention may not contain an electron transport layer (ETL). However, the OLED according to the present invention may optional contain an electron transport layer (ETL).

The electron transport layer is arranged between the emission layer and the organic semiconductor layer according to the invention. The electron transport layer facilitates electron transport from the organic semiconductor layer according to invention into the emission layer. Preferably, the electron transport layer is contacting sandwiched between the emission layer and the organic semi-conductor layer according to the invention. In another preferred embodiment, the electron transport layer is contacting sandwiched between the hole blocking layer and the organic semi-conductor layer according to the invention.

Preferably, the electron transport layer is free of emitter dopants. In another preferred aspect, the electron transport layer is free of metal, metal halide, metal salt and/or lithium organic metal complex.

According to various embodiments the OLED may comprises an electron transport layer or an electron transport layer stack comprising at least a first electron transport layer and at least a second electron transport layer.

According to various embodiments of the OLED of the present invention the electron transport layer may comprises at least one matrix compound. Preferably, the at least one matrix compound is a substantially covalent matrix compound. Further preferred, the matrix compound of the electron transport layer is an organic matrix compound.

It is to be understood that "substantially covalent" means compounds comprising elements bound together mostly by covalent bonds. Substantially covalent matrix material consists of at least one substantially covalent compound. Substantially covalent materials can comprise low molecular weight compounds which may be, preferably, stable enough to be processable by vacuum thermal evaporation (VTE). Alternatively, substantially covalent materials can comprise polymeric compounds, preferably, compounds soluble in a solvent and thus processable in form of a solution. It is to be understood that a polymeric substantially covalent material may be crosslinked to form an infinite irregular network, however, it is supposed that such crosslinked polymeric substantially covalent matrix compounds still comprise both skeletal as well as peripheral atoms. Skeletal atoms of the substantially covalent compound are covalently bound to at least two neighboring atoms.

A compound comprising cations and anions is considered as substantially covalent, if at least the cation or at least the anion comprises at least nine covalently bound atoms.

Preferred examples of substantially covalent matrix compounds are organic matrix compounds consisting predominantly from covalently bound C, H, O, N, S, which may optionally comprise also covalently bound B, P, As, Se. Organometallic compounds comprising covalent bonds carbon-metal, metal complexes comprising organic ligands and metal salts of organic acids are further examples of organic compounds that may serve as organic matrix compounds.

According to a more preferred aspect, the organic matrix compound lacks metal atoms and majority of its skeletal atoms is selected from C, O, S, N

According to a more preferred aspect, the substantially covalent matrix compound comprises a conjugated system of at least six, more preferably at least ten, even more preferably at least fourteen delocalized electrons.

Examples of conjugated systems of delocalized electrons are systems of alternating pi- and sigma bonds. Optionally, one or more two-atom structural units having the pi-bond between its atoms can be replaced by an atom bearing at least one lone electron pair, typically by a divalent atom selected from O, S, Se, Te or by a trivalent atom selected from N, P, As, Sb, Bi. Preferably, the conjugated system of delocalized electrons comprises at least one aromatic or heteroaromatic ring according to the Hückel rule. Also preferably, the substantially covalent matrix compound may comprise at least two aromatic or heteroaromatic rings which are either linked by a covalent bond or condensed.

Preferably the electron transport layer comprises at least a second matrix compound. Suitable matrix compounds are described in EP15201418.9.

According to a more preferred aspect the second organic matrix compound can be an organic matrix compound and selected from the group comprising benzo[k]fluoranthene, pyrene, anthracene, fluorene, spiro(bifluorene), phenanthrene, perylene, triptycene, spiro[fluorene-9,9'-xanthene], coronene, triphenylene, xanthene, benzofurane, dibenzofurane, dinaphthofurane, acridine, benzo[c]acridine, dibenzo[c,h]acridine, dibenzo[a,j]acridine, triazine, pyridine, pyrimidine, carbazole, phenyltriazole, benzimidazole, phenanthroline, oxadiazole, benzooxazole, oxazole, quinazoline, benzo [h]quinazoline, pyrido[3,2-h]quinazoline, pyrimido[4,5-f]quinazoline, quinoline, benzoquinoline, pyrrolo[2,1-a]isoquinolin, benzofuro[2,3-d]pyridazine, thienopyrimidine, dithienothiophene, benzothienopyrimidine, benzothienopyrimidine, phosphine oxide, phosphole, triaryl borane, 2-(benzo[d]oxazol-2-yl)phenoxy metal complex, 2-(benzo[d]thiazol-2-yl)phenoxy metal complex or mixtures thereof.

According to a more preferred aspect there is provided an organic light emitting diode (OLED) wherein the organic light emitting diode comprises at least one electron transport layer comprising at least a second organic matrix compound, wherein the organic semiconductor layer is contacting sandwiched between the first cathode electrode layer and the electron transport layer. The electron transport layer may comprises a second organic matrix compound with a dipole moment of about ≥ 0 Debye and about ≤ 2.5 Debye, preferably ≥ 0 Debye and < 2.3 Debye, more preferably ≥ 0 Debye and < 2 Debye.

According to another aspect there is provided an organic light emitting diode (OLED) wherein the organic light emitting diode comprising at least two electron transport layer of a first electron transport layer and a second electron transport layer. The first electron transport layer may comprises a second organic matrix compound and the second electron transport layer may comprises a third organic matrix compound, wherein the second organic matrix compound of the first electron transport layer may differ from the third organic matrix compound of the second electron transport layer.

According to another embodiment, the dipole moment of the second organic matrix compound may be selected ≥ 0 Debye and ≤ 2.5 Debye, the second organic matrix compound can also be described as non-polar matrix compound.

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule. The dipole moment is determined by a semi-empirical molecular orbital method. The values in Table 5 were calculated using the method as described below. The partial charges and atomic positions are obtained using either the DFT functional of Becke and Perdew BP with a def-SV(P) basis or the hybrid functional B3LYP with a def2-TZVP basis set as implemented in the program package

TURBOMOLE V6.5. If more than one conformation is viable, the conformation with the lowest total energy is selected to determine the dipole moment.

For example, the second organic matrix compound may have a dipole moment between 0 and 2.5 Debye, the first organic matrix compound may contain a center of inversion I, a horizontal mirror plane, more than one $C_n$ axis (n>1), and / or n $C_2$ perpendicular to $C_n$.

If the second organic matrix compound has a dipole moment between 0 and 2.5 Debye, the first organic matrix compound may contain an anthracene group, a pyrene group, a perylene group, a coronene group, a benzo[k]fluoranthene group, a fluorene group, a xanthene group, a dibenzo[c,h]acridine group, a dibenzo[a,j]acridine group, a benzo[c]acridine group, a triaryl borane group, a dithienothiophene group, a triazine group or a benzothienopyrimidine group.

If the second organic matrix compounds has a dipole moment of about ≥ 0 Debye and about ≤ 2.5 Debye, the second organic matrix compound may be free of an imidazole group, a phenanthroline group, a phosphine oxide group, an oxazole group, an oxadiazole group, a triazole group, a pyrimidine group, a quinazoline group, a benzo[h]quinazoline group or a pyrido[3,2-h]quinazoline group.

In a preferred embodiment, the second organic matrix compound is selected from the following compounds or derivatives thereof, the compounds being anthracene, pyrene, coronene, triphenylene, fluorene, spiro-fluorene, xanthene, carbazole, dibenzo[c,h]acridine, dibenzo[a,j]acridine, benzo[c]acridine, triaryl borane compounds, 2-(benzo[d]oxazol-2-yl)phenoxy metal complex; 2-(benzo[d]thiazol-2-yl)phenoxy metal complex, triazine, benzothienopyrimidine, dithienothiophene, benzo[k]fluoranthene, perylene or mixtures thereof.

In a further preferred embodiment, the second organic matrix compound comprises a dibenzo[c,h]acridine compound of formula (2)

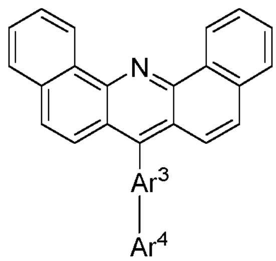

(2)

and/or a dibenzo[a,j]acridine compound of formula (3)

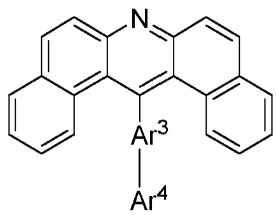

(3)

and/or a benzo[c]acridine compound of formula (4)

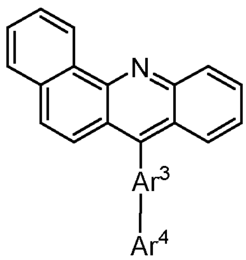

(4)

wherein $Ar^3$ is independently selected from $C_6$-$C_{20}$ arylene, preferably phenylene, biphenylene, or fluorenylene;

$Ar^4$ is independently selected from unsubstituted or substituted $C_6$-$C_{40}$ aryl, preferably phenyl, naphthyl, anthranyl, pyrenyl, or phenanthryl;

and in case that $Ar^4$ is substituted, the one or more substituents may be independently selected from the group consisting of $C_1$-$C_{12}$ alkyl and $C_1$-$C_{12}$ heteroalkyl, wherein $C_1$-$C_5$ alkyl is preferred.

Suitable dibenzo[c,h]acridine compounds are disclosed in EP 2 395 571. Suitable dibenzo[a,j]acridine are disclosed in EP 2 312 663. Suitable benzo[c]acridine compounds are disclosed in WO 2015/083948.

In a further embodiment, it is preferred that the second organic matrix compound comprises a dibenzo[c,h] acridine compound substituted with $C_6$-$C_{40}$ aryl, $C_5$-$C_{40}$ heteroaryl and/or $C_1$-$C_{12}$ alkyl groups, preferably 7-(naphthalen-2-yl)dibenzo[c,h]acridine, 7-(3-(pyren-1-yl)phenyl)dibenzo[c,h]acridine, 7-(3-(pyridin-4-yl)phenyl)dibenzo[c,h]acridine.

In a further embodiment, it is preferred that the second organic matrix compound comprises a dibenzo[a,j] acridine compound substituted with $C_6$-$C_{40}$ aryl, $C_5$-$C_{40}$ heteroaryl and/or $C_1$-$C_{12}$ alkyl groups, preferably 14-(3-(pyren-1-yl)phenyl)dibenzo[a,j]acridine.

In a further embodiment, it is preferred that the second organic matrix compound comprises a benzo[c]acridine compound substituted with $C_6$-$C_{40}$ aryl, $C_5$-$C_{40}$ heteroaryl and/or $C_1$-$C_{12}$ alkyl groups, preferably 7-(3-(pyren-1-yl)phenyl) benzo[c]acridine.

It may be further preferred that the second organic matrix compound comprises a triazine compound of formula (5)

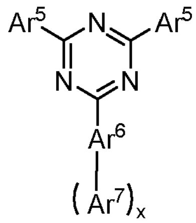

(5)

wherein $Ar^5$ is independently selected from unsubstituted or substituted $C_6$-$C_{20}$ aryl or $Ar^{5.1}$-$Ar^{5.2}$,

wherein $Ar^{5.1}$ is selected from unsubstituted or substituted $C_6$-$C_{20}$ arylene and

$Ar^{5,2}$ is selected from unsubstituted or substituted $C_6$-$C_{20}$ aryl or unsubstituted and substituted $C_5$-$C_{20}$ heteroaryl;

$Ar^6$ is selected from unsubstituted or substituted $C_6$-$C_{20}$ arylene, preferably phenylene, biphenylene, terphenylene, fluorenylene;

$Ar^7$ is independently selected from a group consisting of substituted or unsubstituted aryl, substituted or unsubstituted heteroaryl, the aryl and the heteroaryl having 6 to 40 ring-forming atoms, preferably phenyl, naphthyl, phenantryl, fluorenyl, terphenyl, pyridyl, quinolyl, pyrimidyl, triazinyl, benzo[h]quinolinyl, or benzo[4,5]thieno[3,2-d]pyrimidine;

x is selected from 1 or 2,

wherein in case that $Ar^5$ is substituted the one or more substituents may independently be selected from $C_1$-$C_{12}$ alkyl and $C_1$-$C_{12}$ heteroalkyl, preferably $C_1$-$C_5$ alkyl;

and in case that $Ar^7$ is substituted, the one or more substituents may be independently selected from $C_1$-$C_{12}$ alkyl and $C_1$-$C_{12}$ heteroalkyl, preferably $C_1$-$C_5$ alkyl, and from $C_6$-$C_{20}$ aryl.

Suitable triazine compounds are disclosed in US 2011/284832, WO 2014/171541, WO 2015/008866, WO2015/105313, JP 2015-074649 A, and JP 2015-126140 and KR 2015/0088712.

Furthermore, it is preferred that the second organic matrix compound comprises a triazine compound substituted with $C_6$-$C_{40}$ aryl, $C_5$-$C_{40}$ heteroaryl and/or $C_1$-$C_{12}$ alkyl groups, preferably 3-[4-(4,6-di-2-naphthalenyl-1,3,5-triazin-2-yl) phenyl]quinolone, 2-[3-(6'-methyl[2,2'-bipyridin]-5-yl)-5-(9-phenanthrenyl)phenyl]-4,6-diphenyl-1,3,5-triazine, 2-(3-(phenanthren-9-yl)-5-(pyridin-2-yl)phenyl)-4,6-diphenyl-1,3,5-triazine, 2,4-diphenyl-6-(5'''-phenyl-[1,1':3',1'':3'',1''':3''',1''''-quinquephenyl]-3-yl)-1,3,5-triazine, 2-([1,1'-biphenyl]-3-yl)-4-(3'-(4,6-diphenyl-1,3,5-triazin-2-yl)-[1,1'-biphenyl]-3-yl)-6-phenyl-1,3,5-triazine and/or 2-(3'-(4,6-diphenyl-1,3,5-triazin-2-yl)-[1,1'-biphenyl]-3-yl)-4-phenylbenzo[4,5]thieno[3,2-d]pyrimidine.

Suitable 2-(benzo[d]oxazol-2-yl)phenoxy metal complex or 2-(benzo[d]thiazol-2-yl)phenoxy metal complex are

disclosed in WO 2010/020352..

In a preferred embodiment, the second organic matrix compound comprises a benzothienopyrimidine compound substituted with $C_6$-$C_{40}$ aryl, $C_5$-$C_{40}$ heteroaryl and/or $C_1$-$C_{12}$ alkyl groups, preferably 2-phenyl-4-(4',5',6'-triphenyl-[1,1':2',1":3",1"'-quaterphenyl]-3"'-yl)benzo[4,5]thieno[3,2-d]pyrimidine. Suitable benzothienopyrimidine compounds are disclosed in W 2015/0105316.

In a preferred embodiment, the second organic matrix compound comprises a benzo[k]fluoranthene compound substituted with $C_6$-$C_{40}$ aryl, $C_5$-$C_{40}$ heteroaryl and/or $C_1$-$C_{12}$ alkyl groups, preferably 7, 12-diphenylbenzo[k]fluoranthene. Suitable benzo[k]fluoranthene compounds are disclosed in JP10189247 A2.

In a preferred embodiment, the second organic matrix compound comprises a perylene compound substituted with $C_6$-$C_{40}$ aryl, $C_5$-$C_{40}$ heteroaryl and/or $C_1$-$C_{12}$ alkyl groups, preferably 3,9-bis([1,1'-biphenyl]-2-yl)perylene, 3,9-di(naphthalene-2-yl)perylene or 3,10-di(naphthalene-2-yl)perylene. Suitable perylene compounds are disclosed in US2007202354.

In a preferred embodiment, the second organic matrix compound comprises a pyrene compound. Suitable pyrene compounds are disclosed in US20050025993.

In a preferred embodiment, the second organic matrix compound comprises a spiro-fluorene compound. Suitable spiro-fluorene compounds are disclosed in JP2005032686.

In a preferred embodiment, the second organic matrix compound comprises a xanthene compound. Suitable xanthene compounds are disclosed in US2003168970A and WO 2013149958.

In a preferred embodiment, the second organic matrix compound comprises a coronene compound. Suitable coronene compounds are disclosed in Adachi, C.; Tokito, S.; Tsutsui, T.; Saito, S., Japanese Journal of Applied Physics, Part 2: Letters (1988), 27(2), L269-L271.

In a preferred embodiment, the second organic matrix compound comprises a triphenylene compound. Suitable triphenylene compounds are disclosed in US20050025993.

In a preferred embodiment, the second organic matrix compound is selected from carbazole compounds. Suitable carbazole compounds are disclosed in US2015207079.

In a preferred embodiment, the second organic matrix compound is selected from dithienothiophene compounds. Suitable dithienothiophene compounds are disclosed in KR2011085784.

In a preferred embodiment, the second organic matrix compound comprises an anthracene compound. Particularly preferred are anthracene compounds represented by Formula 400 below:

Formula 400

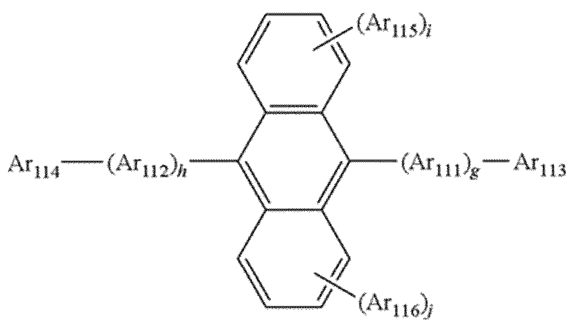

In Formula 400, $Ar_{111}$ and $Ar_{112}$ may be each independently a substituted or unsubstituted $C_6$-$C_{60}$ arylene group; $Ar_{113}$ to $Ar_{116}$ may be each independently a substituted or unsubstituted $C_1$-$C_{10}$ alkyl group or a substituted or unsubstituted $C_6$-$C_{60}$ aryl group; and g, h, i, and j may be each independently an integer from 0 to 4.

In some embodiments, $Ar_{111}$ and $Ar_{112}$ in Formula 400 may be each independently one of

a phenylene group, a naphthylene group, a phenanthrenylene group, or a pyrenylene group; or

a phenylene group, a naphthylene group, a phenanthrenylene group, a fluorenyl group, or a pyrenylene group, each substituted with at least one of a phenyl group, a naphthyl group, or an anthryl group.

In Formula 400, g, h, i, and j may be each independently an integer of 0, 1, or 2.

In Formula 400, $Ar_{113}$ to $Ar_{116}$ may be each independently one of

a $C_1$-$C_{10}$ alkyl group substituted with at least one of a phenyl group, a naphthyl group, or an anthryl group;

a phenyl group, a naphthyl group, an anthryl group, a pyrenyl group, a phenanthrenyl group, or a fluorenyl group;

a phenyl group, a naphthyl group, an anthryl group, a pyrenyl group, a phenanthrenyl group, or a fluorenyl group, each substituted with at least one of a deuterium atom, a halogen atom, a hydroxyl group, a cyano group, a nitro group, an amino group, an amidino group, a hydrazine group, a hydrazone group, a carboxyl group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a phenyl group, a naphthyl group, an anthryl group, a pyrenyl group, a phenanthrenyl group, or a fluorenyl group; or

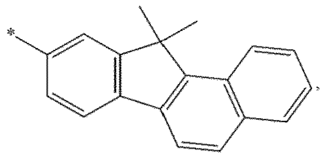

but embodiments of the invention are not limited thereto.

In another aspect, the electron transport layer may comprise a polar second organic matrix compound. Preferably, the second organic matrix compound has a dipole moment of about > 2.5 Debye and < 10 Debye, preferably > 3 and < 5 Debye, even more preferred > 2.5 and less than 4 Debye.

If an organic matrix compounds has a dipole moment of > 2.5 and < 10 Debye, the organic matrix compound may be described by one of the following symmetry groups: $C_1$, $C_n$, $C_{nv}$, or $C_s$.

When an organic matrix compound has a dipole moment of > 2.5 and < 10 Debye, the organic matrix compound may comprise benzofurane, dibenzofurane, dinaphthofurane, pyridine, acridine, phenyltriazole, benzimidazole, phenanthroline, oxadiazole, benzooxazole, oxazole, quinazoline, benzoquinazoline, pyrido[3,2-h]quinazoline, pyrimido[4,5-f]quinazoline, quinoline, benzoquinoline, pyrrolo[2,1-a]isoquinolin, benzofuro[2,3-d]pyridazine, thienopyrimidine, phosphine oxide, phosphole or mixtures thereof.

It is further preferred that the second organic matrix compound comprises a phosphine oxide compound substituted with $C_6$-$C_{40}$ aryl, $C_5$-$C_{40}$ heteroaryl and/or $C_1$-$C_{12}$ alkyl groups, preferably (3-(dibenzo[c,h]acridin-7-yl)phenyl)diphenylphosphine oxide, 3-phenyl-3H-benzo[b]dinaphtho[2,1-d:1',2'- f]phosphepine-3-oxide, phenyldi(pyren-1-yl)phosphine oxide, bis(4-(anthracen-9-yl)phenyl)(phenyl)phosphine oxide, (3-(9,10-di(naphthalen-2-yl)anthracen-2-yl)phenyl)diphenylphosphine oxide, phenyldi(pyren-1-yl)phosphine oxide, diphenyl(5-(pyren-1-yl)pyridin-2-yl)phosphine oxide, diphenyl(4'-(pyren-1-yl)-[1,1'-biphenyl]-3-yl)phosphine oxide, diphenyl(4'-(pyren-1-yl)-[1,1'-biphenyl]-3-yl)phosphine oxide, (3'-(dibenzo[c,h]acridin-7-yl)-[1,1'-biphenyl]-4-yl)diphenylphosphine oxide and/or phenyl bis(3-(pyren-1-yl)phenyl)phosphine oxide.

Diarylphosphine oxide compounds which may be used as second organic matrix compound are disclosed in EP 2395571 A1, WO2013079217 A1, EP 13187905, EP13199361 and JP2002063989 A1. Dialkylphosphine oxide compounds are disclosed in EP15195877.4.

It is further preferred that the second organic matrix compound comprises a benzimidazole compound substituted with $C_6$-$C_{40}$ aryl, $C_5$-$C_{40}$ heteroaryl and/or $C_1$-$C_{12}$ alkyl groups, preferably 2-(4-(9,10-di(naphthalen-2-yl)anthracene-2-yl)phenyl)-1-phenyl-1H-benzo[d]imidazole, 1-(4-(10-([1,1'-biphenyl]-4-yl)anthracen-9-yl)phenyl)-2-ethyl-1H-benzo[d]imidazole, and/or 1,3,5-tris(1-phenyl-1H-benzimidazol-2-yl)benzene.

Benzimidazole compounds that can be used as second organic matrix materials are disclosed in US 6878469 and WO2010134352.

In a preferred embodiment, the second organic matrix compound comprises a quinoline compound. Suitable quinoline compounds are disclosed in US 20090108746 and US 20090166670.

In a preferred embodiment, the second organic matrix matrix compound comprises a benzoquinoline compound. Suitable benzoquinoline compounds are disclosed in JP 2004281390 and US 20120280613.

In a preferred embodiment, the second organic matrix compound comprises a pyrimidine compound. Suitable pyrimidine compounds are disclosed in JP2004031004.

In a preferred embodiment, the second organic matrix compound comprises an oxazole compound. Preferred oxazole compounds are disclosed in JP2003007467 and WO2014163173.

In a preferred embodiment, the second organic matrix compound comprises an oxadiazole compound. Preferred oxadiazole compounds are disclosed in US2015280160.

In a preferred embodiment, the second organic matrix compound comprises an benzooxazole compound. Preferred benzooxazole compounds are disclosed in Shirota and Kageyama, Chem. Rev. 2007, 107, 953-1010.

In a preferred embodiment, the second organic matrix compound comprises a triazole compound. Suitable triazole compounds are disclosed in US2015280160.

In a preferred embodiment, the second organic matrix compound comprises a pyrimido[4,5-flquinazoline compound. Suitable pyrimido[4,5-f]quinazoline compounds are disclosed in EP2504871.

In a preferred embodiment, the second organic matrix compound may be selected from the group consisting of a compound represented by Formula 2, and a compound represented by Formula 3 below:

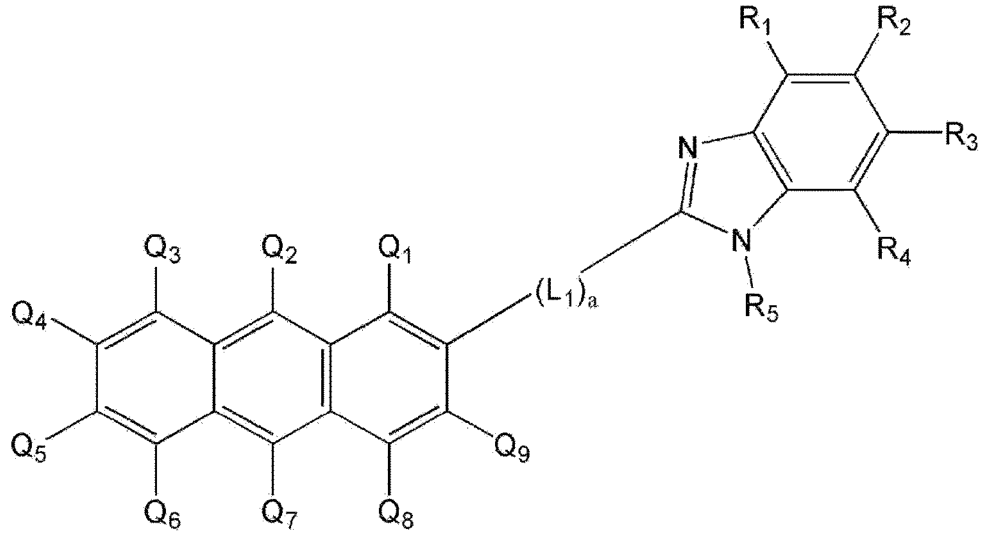

Formula 2

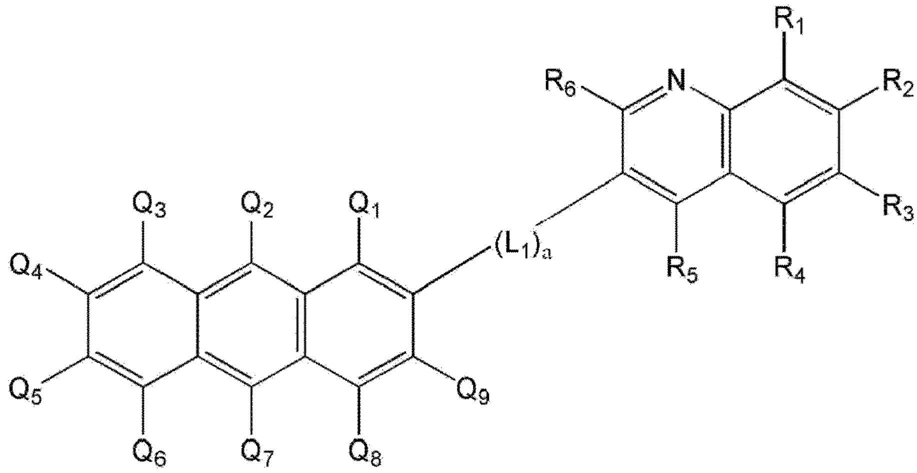

Formula 3

In Formulae 2 and 3, $R_1$ to $R_6$ are each independently a hydrogen atom, a halogen atom, a hydroxy group, a cyano group, a substituted or unsubstituted $C_1$-$C_{30}$ alkyl group, a substituted or unsubstituted $C_1$-$C_{30}$ alkoxy group, a substituted or unsubstituted $C_1$-$C_{30}$ acyl group, a substituted or unsubstituted $C_2$-$C_{30}$ alkenyl group, a substituted or unsubstituted $C_2$-$C_{30}$ alkynyl group, a substituted or unsubstituted $C_6$-$C_{30}$ aryl group, or a substituted or unsubstituted $C_3$-$C_{30}$ heteroaryl group. At least two adjacent $R_1$ to $R_6$ groups are optionally bonded to each other, to form a saturated or unsaturated ring. $L_1$ is a bond, a substituted or unsubstituted $C_1$-$C_{30}$ alkylene group, a substituted or unsubstituted $C_6$-$C_{30}$ arylene group, or a substituted or unsubstituted $C_3$-$C_{30}$ hetero arylene group. $Q_1$ through $Q_9$ are each independently a hydrogen atom, a substituted or unsubstituted $C_6$-$C_{30}$ aryl group, or a substituted or unsubstituted $C_3$-$C_{30}$ hetero aryl

group, and "a" is an integer from 1 to 10.

For example, $R_1$ to $R_6$ may be each independently selected from the group consisting of a hydrogen atom, a halogen atom, a hydroxy group, a cyano group, a methyl group, an ethyl group, a propyl group, a butyl group, a methoxy group, an ethoxy group, a propoxy group, a butoxy group, a phenyl group, a naphthyl group, an anthryl group, a pyridinyl group, and a pyrazinyl group.

In particular, in Formula 2 and/or 3, $R_1$ to $R_4$ may each be a hydrogen atom, $R_5$ may be selected from the group consisting of a halogen atom, a hydroxy group, a cyano group, a methyl group, an ethyl group, a propyl group, a butyl group, a methoxy group, an ethoxy group, a propoxy group, a butoxy group, a phenyl group, a naphthyl group, an anthryl group, a pyridinyl group, and a pyrazinyl group. In addition, in Formula 3, $R_1$ to $R_6$ may each be a hydrogen atom.

For example, in Formula 2 and/or 3, $Q_1$ to $Q_9$ are each independently a hydrogen atom, a phenyl group, a naphthyl group, an anthryl group, a pyridinyl group, and a pyrazinyl group. In particular, in Formulae 2 and/or 3, $Q_1$, $Q_3$-$Q_6$, $Q_8$ and $Q_9$ are hydrogen atoms, and $Q_2$ and $Q_7$ may be each independently selected from the group consisting of a phenyl group, a naphthyl group, an anthryl group, a pyridinyl group, and a pyrazinyl group.

For example, $L_1$, in Formula 2 and/or 3, may be selected from the group consisting of a phenylene group, a naphthylene group, an anthrylene group, a pyridinylene group, and a pyrazinylene group. In particular, $L_1$ may be a phenylene group or a pyridinylene group. For example, "a" may be 1, 2, or, 3.

The second organic matrix compound may be further selected from Compound 5, 6, or 7 below:

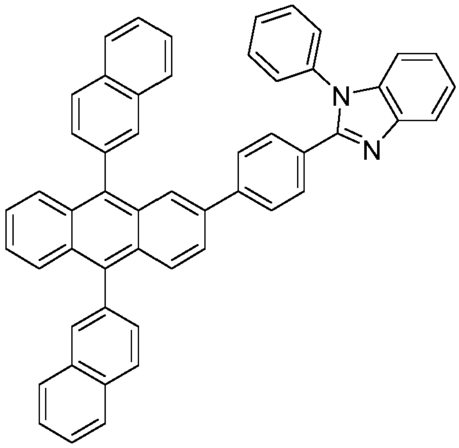

Compound 6

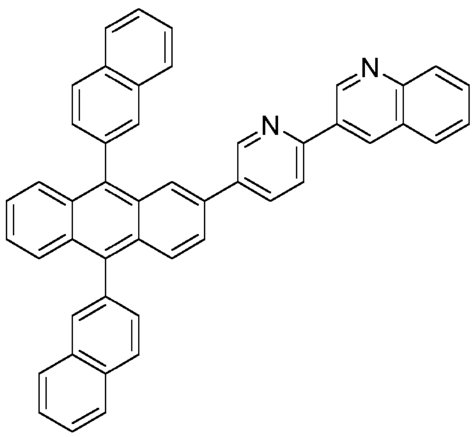

Compound 7.

Preferably, the second organic matrix compound comprises a phenanthroline compound substituted with $C_6$-$C_{40}$ aryl, $C_5$-$C_{40}$ heteroaryl and/or $C_1$-$C_{12}$ alkyl groups, preferably 2,4,7,9-tetraphenyl-1,10-phenanthroline, 4,7-diphenyl-2,9-di-p-tolyl-1,10-phenanthroline, 2,9-di(biphenyl-4-yl)-4,7-diphenyl-1,10-phenanthroline and/or 3,8-bis(6-phenyl-2-pyridinyl)- 1,10-phenanthroline.

Phenanthroline compounds that can be used as second organic matrix materials are disclosed in EP 1786050 A1 and CN102372708.

Other suitable second organic matrix compounds that can be used are quinazoline compounds substituted with aryl or heteroaryl groups, preferably 9-phenyl-9'-(4-phenyl-2-quinazolinyl)- 3,3'-bi-9H-carbazole. It is further preferred that the first organic matrix compound comprises a quinazoline compound substituted with $C_6$-$C_{40}$ aryl, $C_5$-$C_{40}$ heteroaryl and/or $C_1$-$C_{12}$ alkyl groups, preferably 9-phenyl-9'-(4-phenyl-2-quinazolinyl)- 3,3'-bi-9*H*-carbazole. Quinazoline compounds that can be used as first organic matrix materials are disclosed in KR2012102374.

It is further preferred that the second organic matrix compound comprises a benzo[h]quinazoline compound substituted with $C_6$-$C_{40}$ aryl, $C_5$-$C_{40}$ heteroaryl and/or $C_1$-$C_{12}$ alkyl groups, preferably 4-(2-naphthalenyl)-2-[4-(3-quinolinyl)phenyl]- benzo[h]quinazoline. Benzo[h]quinazoline compounds that can be used as first organic matrix materials are disclosed in KR2014076522.

It is also preferred that the second organic matrix compound comprises a pyrido[3,2-h]quinazoline compound substituted with $C_6$-$C_{40}$ aryl, $C_5$-$C_{40}$ heteroaryl and/or $C_1$-$C_{12}$ alkyl groups, preferably 4-(naphthalen-1-yl)-2,7,9-triphenylpyrido[3,2-h]quinazoline. Pyrido[3,2-h]quinazoline compounds that can be used as first organic matrix materials are disclosed in EP1970371.

In a further preferred embodiment, the second organic matrix compound is selected from acridine compounds.

Suitable acridine compounds are disclosed in CN104650032.

According to another aspect, the electron transport layer can be in direct contact with the organic semiconductor layer according to the invention. If more than one electron transport layer is present, the organic semiconductor layer is contacting sandwiched between the first electron transport layer and the first cathode electrode layer. The second electron transport layer, if present, is contacting sandwiched between the emission layer and the first electron transport layer.

According to various embodiments of the OLED of the present invention the thicknesses of the electron transport layer may be in the range of about ≥ 0.5 nm to about ≤ 95 nm, preferably of about ≥ 3 nm to about ≤ 80 nm, further preferred of about ≥ 5 nm to about ≤ 60 nm, also preferred of about ≥ 6 nm to about ≤ 40 nm, in addition preferred about ≥ 8 nm to about ≤ 20 nm and more preferred of about ≥ 10 nm to about ≤ 18 nm.

According to various embodiments of the OLED of the present invention the thicknesses of the electron transport layer stack can be in the range of about ≥ 25 nm to about ≤ 100 nm, preferably of about ≥ 30 nm to about ≤ 80 nm, further preferred of about ≥ 35 nm to about ≤ 60 nm, and more preferred of about ≥ 36 nm to about ≤ 40 nm.

The ETL may be formed optional on an EML or on the HBL if the HBL is formed.

The ETL may have a stacked structure, preferably of two ETL-layers, so that injection and transport of electrons may be balanced and holes may be efficiently blocked. In a conventional OLED, since the amounts of electrons and holes vary with time, after driving is initiated, the number of excitons generated in an emission area may be reduced. As a result, a carrier balance may not be maintained, so as to reduce the lifetime of the OLED.

However, in the ETL, the first layer and the second layer may have similar or identical energy levels, so that the carrier balance may be uniformly maintained, while controlling the electron-transfer rate.

The organic light emitting device may comprise further electron transport layers, preferably a third and optional fourth electron transport layer, wherein the third and optional fourth electron transport layer is arranged between the charge generation layer and the cathode. Preferably, the first electron transport layer and third electron transport layer are selected the same, and the second and fourth electron transport layer are selected the same.

The ETL may be formed on the EML by vacuum deposition, spin coating, slot-die coating, printing, casting, or the like. When the ETL is formed by vacuum deposition or spin coating, the deposition and coating conditions may be similar to those for formation of the HIL. However, the deposition and coating conditions may vary, according to a compound that is used to form the ETL.

In another embodiment, the ETL may contain an alkali organic complex and / or alkali halide, preferably a lithium organic complex and / or lithium halide.

According to various aspects the lithium halide can be selected from the group comprising LiF, LiCl, LiBr or LiJ, and preferably LiF.

According to various aspects the alkali organic complex can be a lithium organic complex and preferably the lithium organic complex can be selected from the group comprising a lithium quinolate, a lithium borate, a lithium phenolate, a lithium pyridinolate or a lithium Schiff base and lithium fluoride, preferably a lithium 2-(diphenylphosphoryl)-phenolate, lithium tetra(1H-pyrazol-1-yl)borate, a lithium quinolate of formula (III), a lithium 2-(pyridin-2-yl)phenolate and LiF, and more preferred selected from the group comprising a lithium 2-(diphenylphosphoryl)-phenolate, lithium tetra(1H-pyrazol-1-yl)borate, a lithium quinolate of formula (III) and a lithium 2-(pyridin-2-yl)phenolate.

More preferred, the alkali organic complex is a lithium organic complex and/or the alkali halide is lithium halide.

Suitable lithium organic complexes are described in WO2016001283A1.

## Charge generation layer

Charge generation layers (CGL) that can be suitable used for the OLED of the present invention are described in US 2012098012 A.

The charge generation layer is generally composed of a double layer. The charge generation layer can be a pn junction charge generation layer joining n-type charge generation layer and p-type charge generation layer. The pn junction charge generation layer generates charges or separates them into holes and electrons; and injects the charges into the individual light emission layer. In other words, the n-type charge generation layer provides electrons for the first light emission layer adjacent to the anode electrode while the p-type charge generation layer provides holes to the second light emission layer adjacent to the cathode electrode, by which luminous efficiency of an organic light emitting device incorporating multiple light emission layers can be further improved and at the same time, driving voltage can be lowered.

The n-type charge generation layer can be composed of metal or organic material doped with n-type. The metal can be one selected from a group consisting of Li, Na, K, Rb, Cs, Mg, Ca, Sr, Ba, La, Ce, Sm, Eu, Tb, Dy, and Yb. Also, n-type dopant and host used for organic material doped with the n-type can employ conventional materials. For example, the n-type dopant can be alkali metal, alkali metal compound, alkali earth metal, or alkali earth metal compound. More specifically, the n-type dopant can be one selected from a group consisting of Cs, K, Rb, Mg, Na, Ca, Sr, Eu and Yb. The host material can be one selected from a group consisting of tris(8-hydroxyquinoline)aluminum, triazine, hydroxyquinoline derivative, benzazole derivative, and silole derivative.

The p-type charge generation layer can be composed of metal or organic material doped with p-type dopant. Here, the metal can be one or an alloy consisting of two or more selected from a group consisting of Al, Cu, Fe, Pb, Zn, Au, Pt, W, In, Mo, Ni, and Ti. Also, p-type dopant and host used for organic material doped with the p-type can employ conventional materials. For example, the p-type dopant can be one selected from a group consisting of tetrafluore-7,7,8,8-tetracyanoquinodimethane (F4-TCNQ), derivative of tetracyanoquinodimethane, radialene derivative, iodine, FeCl3, FeF3, and SbC15. Preferably, the p-type dopant is selected from radialene derivatives. The host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB).

In another embodiment, the p-type charge generation layer is arranged adjacent to the organic semiconductor layer. The p-type charge generation layer according to one embodiment may include compounds of the following Chemical Formula 16.

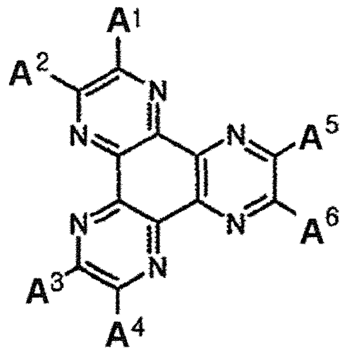

(16),

wherein

each of $A^1$ to $A^6$ may be hydrogen, a halogen atom, nitrile (-CN), nitro (-$NO_2$), sulfonyl (-$SO_2$R), sulfoxide (-SOR), sulfonamide (-$SO_2$NR), sulfonate (-$SO_3$R), trifluoromethyl (-$CF_3$), ester (-COOR), amide (-CONHR or - CONRR'), substituted or unsubstituted straight-chain or branched-chain C1-C12 alkoxy, substituted or unsubstituted straight-chain or branched-chain C1-C12 alkyl, substituted or unsubstituted straight-chain or branched chain C2-C12 alkenyl, a substituted or unsubstituted aromatic or non-aromatic heteroring, substituted or unsubstituted aryl, substituted or unsubstituted mono- or di-arylamine, substituted or unsubstituted aralkylamine, or the like.

Herein, each of the above R and R' may be substituted or unsubstituted $C_1$-$C_{60}$ alkyl, substituted or unsubstituted aryl, or a substituted or unsubstituted 5- to 7-membered heteroring, or the like.

Particularly preferred is an p-type charge generation layer comprising a compound of Formula (17)

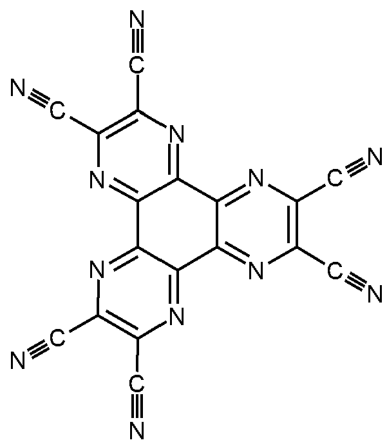

(17).

The p-type charge generation layer is arranged on top of the n-type charge generation layer. As the materials for the p-type charge generation layer, aryl amine-based compounds may be used. One embodiment of the aryl amine-based compounds includes compounds of the following Chemical Formula 18:

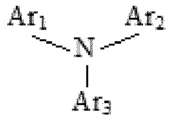

(18),

wherein

$Ar_1$, $Ar_2$ and $Ar_3$ are each independently hydrogen or a hydrocarbon group. Herein, at least one of $Ar_1$, $Ar_2$ and $Ar_3$ may include aromatic hydrocarbon substituents, and each substituent may be the same, or they may be composed of different substituents. When $Ar_1$, $Ar_2$ and $Ar_3$ are not aromatic hydrocarbons, they may be hydrogen; a straight-chain, branched-chain or cyclic aliphatic hydrocarbon; or a heterocyclic group including N, O, S or Se.

In another aspect, an organic light emitting diode of the present invention is provided, wherein the organic light emitting diode further comprises a p-type charge generation layer, wherein the organic semiconductor layer is arranged between the first emission layer and the p-type charge generation layer. Preferably, the p-type charge generation layer comprises, more preferably consists of, a radialene dopant and a host.

In another embodiment, the p-type charge generation layer is in direct contact with the organic semiconductor layer of the present invention. Preferably, the p-type charge generation layer comprising or consisting of a radialene dopant and a host is in direct contact with the organic semi-conductor layer.

In another aspect an organic light emitting diode of the present invention is provided which further comprising a p-type charge generation layer, wherein the p-type charge generation layer is arranged between the organic semiconductor layer and the cathode electrode. If the cathode electrode is transparent to visible light emission, this arrangement may enable efficient electron injection into the emission layer.

Organic light emitting diode (OLED)

According to another aspect of the present invention, there is provided an organic light emitting diode (OLED) comprising: a substrate, an anode electrode a first hole injection layer, a first hole transport layer, optional first electron blocking layer, a first emission layer, optional a first hole blocking layer, optional a first electron transport layer, optional an organic semiconductor layer of the present invention, an n-type charge generation layer, a p-type charge generation layer, a second hole transport layer, optional second electron blocking layer a second emission layer, optional a second hole blocking layer, optional a second electron transport layer, the organic semiconductor layer, optional an electron injection layer and a cathode electrode layer, wherein the layers are arranged in that order.

According to various embodiments of the OLED of the present invention, the OLED may not comprises an electron transport layer.

According to various embodiments of the OLED of the present invention, the OLED may not comprises an electron blocking layer.

According to various embodiments of the OLED of the present invention, the OLED may not comprises a hole blocking layer.

According to various embodiments of the OLED of the present invention, the OLED may not comprises a second emission layer.

Electronic device

Another aspect is directed to an electronic device comprising at least one organic light-emitting diode (OLED). A device comprising organic light emitting diodes (OLED) is for example a display or a lighting panel.

Additional aspects and/or advantages of the invention will be set forth in part in the description which follows and, in part, will be obvious from the description, or may be learned by practice of the invention.

Method of manufacture

As mentioned before, the invention relates to a method of manufacturing an inventive organic light emitting diode comprising the steps of sequentially forming an anode electrode, at least one emission layer, at least one organic semiconductor layer, and a cathode electrode on a substrate, and forming the at least one organic semiconductor layer by co-depositing the substantially metallic zero valent rare earth metal dopant selected from Sm, Eu, and Yb together with a first matrix compound comprising phenanthrolinyl groups.

However, it is also in accordance with the invention that the organic light emitting diode is manufactured by sequentially forming a cathode electrode on a substrate, at least one organic semiconductor layer, at least one emission layer and an anode electrode, wherein, again, the at least one organic semiconductor layer is formed by co-depositing the substantially metallic zero valent rare earth metal dopant selected from Sm, Eu, and Yb together with a first matrix

compound comprising two phenanthrolinyl groups.

The term co-deposition in this regard is particularly related to depositing the substantially metallic rare earth metal dopant from a first evaporation source and the first matrix compound, preferably from a second evaporation source.

Surprisingly, rare earth metal dopants can be co-deposited with organic matrix compounds. This is very difficult to achieve for alkali metals, in particular Li, as the doping concentration is very low compared to rare earth metal dopants. Therefore, alkali metals are typically deposited after the organic matrix compound.

In another embodiment, the organic semiconductor layer is formed by co-depositing th substantially metallic zero valent rare earth metal dopant selected from Sm, Eu, and Yb together with a first matrix compound comprising two phenanthrolinyl groups in the same evaporation chamber.

According to another embodiment the method comprises a further step of depositing an electron transport layer on the emission layer. In this case, it is clear that the organic semiconductor layer is deposited on the electron transport layer instead.

Depositing in terms of the invention may be achieved by depositing via vacuum thermal evaporation or depositing via solution processing, preferably, the processing being selected from spin-coating, printing, casting and/or slot-die coating.

It is preferred that depositing the organic semiconductor layer comprises vacuum thermal evaporation.

According to various embodiments of the present invention, the method may further include forming on a substrate an anode electrode a first hole injection layer, a first hole transport layer, optional first electron blocking layer, a first emission layer, optional a first hole blocking layer, optional a first electron transport layer, the organic semiconductor layer of the present invention, an p-type charge generation layer, a second hole transport layer, optional second electron blocking layer, a second emission layer, optional a second hole blocking layer, optional a second electron transport layer, the organic semiconductor layer, and a cathode electrode layer, wherein the layers are arranged in that order; or the layers are deposited the other way around, starting with the cathode electrode layer; and more preferred the organic semiconductor layer is be deposited before the cathode electrode layer is deposited.

However, according to one aspect the layers are deposited the other way around, starting with the cathode electrode, and sandwiched between the cathode electrode and the anode electrode.

The anode electrode and/or the cathode electrode can be deposited on a substrate. Preferably the anode is deposited on a substrate.

According to another aspect of the present invention, there is provided a method of manufacturing an organic light-emitting diode (OLED), the method using:

- at least one deposition source, preferably two deposition sources and more preferred at least three deposition sources; and/or

- deposition via vacuum thermal evaporation (VTE); and/or

- deposition via solution processing, preferably the processing is selected from spin-coating, printing, casting and/or slot-die coating.

## BRIEF DESCRIPTION OF THE DRAWINGS

These and/or other aspects and advantages of the present invention will become apparent and more readily appreciated from the following description of the exemplary embodiments, taken in conjunction with the accompanying drawings, of which:

FIG. 1 is a schematic sectional view of an organic light-emitting diode (OLED);
FIG. 2 is a schematic sectional view of an OLED.
FIG. 3 is a schematic sectional view of an OLED.
FIG. 4 is a schematic sectional view of a tandem OLED comprising a charge generation layer.
FIG. 5 is a schematic sectional view of an OLED comprising a charge generation layer in direct contact with the cathode electrode, according to an exemplary embodiment of the present invention.

## DETAILED DESCRIPTION

Reference will now be made in detail to the exemplary embodiments of the present invention, examples of which are illustrated in the accompanying drawings, wherein like reference numerals refer to the like elements throughout. The exemplary embodiments are described below, in order to explain the aspects of the present invention, by referring to the figures.

Herein, when a first element is referred to as being formed or disposed "on" a second element, the first element can be disposed directly on the second element, or one or more other elements may be disposed there between. When a first element is referred to as being formed or disposed "directly on" a second element, no other elements are disposed there between.

FIG. 1 is a schematic sectional view of an organic light-emitting diode (OLED) 100. The OLED 100 includes a substrate 110, an anode electrode 120, a hole injection layer (HIL) 130, a hole transport layer (HTL) 140, an emission layer (EML) 150. Onto the emission layer (EML) 150 the organic semiconductor layer 170 is disposed. The organic semiconductor layer 170 comprising or consisting of a substantially metallic rare earth metal dopant and a first matrix compound comprising at least two phenanthrolinyl groups, preferably comprising formula 1, is formed directly on the EML 150. The cathode electrode layer 190 is disposed directly onto the organic semiconductor layer 170.

Fig. 2 is a schematic sectional view of an OLED 100. Fig. 2 differs from Fig. 1 in that the OLED 100 of Fig. 2 comprises an electron transport layer 160.

Referring to Fig. 2 the OLED 100 includes a substrate 110, an anode electrode 120, a hole injection layer (HIL) 130, a hole transport layer (HTL) 140, an emission layer (EML) 150. Onto the emission layer (EML) 150 an electron transport layer (ETL) 160 is disposed. Onto the electron transport layer (ETL) 160 the organic semiconductor layer 170 is disposed. The organic semiconductor layer 170 comprising or consisting of a substantially metallic rare earth metal dopant and a first matrix compound comprising at least two phenanthrolinyl groups,, preferably comprising of formula 1 is formed directly on the ETL 160. The cathode electrode layer 190 is disposed directly onto the organic semiconductor layer 170.

Fig. 3 is a schematic sectional view of an OLED 100. Fig. 3 differs from Fig. 2 in that the OLED 100 of Fig. 3 comprises an electron blocking layer (EBL) 145 and a cathode electrode 190 comprising a first cathode layer 191 and a second cathode layer 192.

Referring to Fig. 3 the OLED 100 includes a substrate 110, an anode electrode 120, a hole injection layer (HIL) 130, a hole transport layer (HTL) 140, an electron blocking layer (EBL) 145 and an emission layer (EML) 150. Onto the emission layer (EML) 150 an electron transport layer (ETL) 160 is disposed. Onto the electron transport layer (ETL) 160 the organic semiconductor layer 170 is disposed. The organic semiconductor layer 170 comprising or consisting of a substantially metallic rare earth metal dopant and a first matrix compound comprising at least two phenanthrolinyl groups,, preferably comprising of formula 1 is formed directly on the ETL 160. The cathode electrode layer 190 comprises of a first cathode layer 191 and a second cathode layer 191. The first cathode layer 191 is a substantially metallic layer and it is disposed directly onto the organic semiconductor layer 170. The second cathode layer 192 is disposed directly onto the first cathode layer 191.

Fig. 4 is a schematic sectional view of a tandem OLED 100n. Fig. 4 differs from Fig. 2 in that the OLED 100 of Fig. 4 further comprises a charge generation layer and a second emission layer.

Referring to Fig. 4 the OLED 100 includes a substrate 110, an anode electrode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a p-type charge generation layer (p-type GCL) 135, a second hole transport layer (HTL) 141, a second electron blocking layer (EBL) 146, a second emission layer (EML) 151, a second hole blocking layer (EBL) 156, a second electron transport layer (ETL) 161, the organic semiconductor layer 170, a first cathode electrode layer 191 and a second cathode electrode layer 192. The organic semiconductor layer 170 comprising or consisting of a substantially metallic rare earth metal dopant and a first matrix compound comprising at least two phenanthrolinyl groups,, preferably comprising of formula 1, is disposed directly onto the second electron transport layer 161 and the first cathode electrode layer 191 is disposed directly onto the organic semiconductor layer 170. The second cathode electrode layer 192 is disposed directly onto the first cathode electrode layer 191. Optionally, the n-type charge generation layer (n-type CGL) 185 may be the organic semiconductor layer of the present invention.

Fig. 5 is a schematic sectional view of an OLED 100, according to an exemplary embodiment of the present invention. Fig. 5 differs from Fig. 1 in that the OLED 100 of Fig. 5 further comprises a p-type charge generation layer in direct contact with the cathode electrode.

Referring to Fig. 5, the OLED 100 includes a substrate 110, an anode electrode 120, a hole injection layer (HIL) 130, a hole transport layer (HTL) 140, an emission layer (EML) 150. Onto the emission layer (EML) 150 the organic semiconductor layer 170 is disposed. The organic semiconductor layer 170 comprising or consisting of a substantially metallic rare earth metal dopant selected from Sm, Eu, and Yb and a first matrix compound comprising two phenanthrolinyl groups, preferably comprising formula 1, is formed directly on the EML 150. The p-type charge generation layer (p-type CGL) 135 is formed directly on the organic semiconductor layer of the present invention 170. The cathode electrode layer 190 is disposed directly onto the p-type charge generation layer 135.

In the description above the method of manufacture an OLED 100 of the present invention is started with a substrate 110 onto which an anode electrode 120 is formed, on the anode electrode 120, a first hole injection layer 130, first hole transport layer 140, optional a first electron blocking layer 145, a first emission layer 150, optional a first hole blocking layer 155, optional an ETL 160, an n-type CGL 185, a p-type CGL 135, a second hole transport layer 141, optional a second

electron blocking layer 146, a second emission layer 151, an optional second hole blocking layer 156, an optional at least one second electron transport layer 161, the organic semiconductor layer 170, a first cathode electrode layer 191 and an optional second cathode electrode layer 192 are formed, in that order or the other way around.

While not shown in Fig. 1, Fig. 2, Fig. 3, Fig. 4 and Fig. 5, a sealing layer may further be formed on the cathode electrodes 190, in order to seal the OLEDs 100. In addition, various other modifications may be applied thereto.

## EXAMPLES

First matrix compounds comprising at least two phenanthrolinyl groups can be synthesized as described in JP2002352961.

### Bottom emission devices with an evaporated emission layer

For bottom emission devices - Examples 1 to 3 and comparative examples 1 to 5, a 15Ω /cm 2 glass substrate (available from Corning Co.) with 90 nm ITO was cut to a size of 50 mm x 50 mm x 0.7 mm, ultrasonically cleaned with isopropyl alcohol for 5 minutes and then with pure water for 5 minutes, and cleaned again with UV ozone for 30 minutes, to prepare a first electrode.

Then, 97 wt.-% of Biphenyl-4-yl(9,9-diphenyl-9H-fluoren-2-yl)-[4-(9-phenyl-9H-carbazol-3-yl)phenyl]-amine (CAS 1242056-42-3) and 3 wt.-% of 2,2',2"-(cyclopropane-1,2,3-triylidene)tris(2-(p-cyanotetrafluorophenyl)acetonitrile) was vacuum deposited on the ITO electrode, to form a HIL having a thickness of 10 nm. Then Biphenyl-4-yl(9,9-diphenyl-9H-fluoren-2-yl)-[4-(9-phenyl-9H-carbazol-3-yl)phenyl]-amine was vacuum deposited on the HIL, to form a HTL having a thickness of 130 nm. 97 wt.-% of ABH113 (Sun Fine Chemicals) as a host and 3 wt.-% of NUBD370 (Sun Fine Chemicals) as a dopant were deposited on the HTL, to form a blue-emitting EML with a thickness of 20 nm.

Then, the organic semiconductor layer is formed by deposing a matrix compound and metal dopant according to examples 1 to 3 and comparative example 1 and 5 by deposing the matrix compound from a first deposition source and rare earth metal dopant from a second deposition source directly on the EML. The composition of the organic semiconductor layer can be seen in Table 1. In examples 1 to 3 the matrix compound is a compound of formula 1. The thickness of the organic semiconductor layer is 36 nm.

Then, the cathode electrode layer is formed by evaporating and/ or sputtering the cathode material at ultra-high vacuum of $10^{-7}$ bar and deposing the cathode layer directly on the organic semiconductor layer. A thermal single co-evaporation or sputtering process of one or several metals is performed with a rate of 0, 1 to 10 nm/s (0.01 to 1 Å/s) in order to generate a homogeneous cathode electrode with a thickness of 5 to 1000 nm. The thickness of the cathode electrode layer is 100 nm. The composition of the cathode electrode can be seen in Table 1. Al and Ag are evaporated while ITO is sputtered onto the organic semiconductor layer using a RF magnetron sputtering process.

### Bottom emission devices with a solution-processed emission layer

For bottom emission devices, a 15Ω /cm 2 glass substrate (available from Corning Co.) with 100 nm Ag was cut to a size of 50 mm x 50 mm x 0.7 mm, ultrasonically cleaned with isopropyl alcohol for 5 minutes and then with pure water for 5 minutes, and cleaned again with UV ozone for 30 minutes, to prepare a first electrode.

Then, PEDOT:PSS (Clevios P VP AI 4083) is spin-coated directly on top of the first electrode to form a 55 nm thick HIL. The HIL is baked on hotplate at 150 °C for 5 min. Then, a light-emitting polymer, for example MEH-PPV, is spin-coated directly on top of the HIL to form a 40 nm thick EML. The EML is baked on a hotplate at 80 °C for 10 min. The device is transferred to an evaporation chamber and the following layers are deposited in high vacuum.

First matrix compound comprising at least two phenanthrolinyl groups and rare earth metal dopant are deposed directly on top of the EML to form the organic semiconductor layer with a thickness of 4 nm. A cathode electrode layer is formed by deposing a 100 nm thick layer of aluminium directly on top of the organic semiconductor layer.

### Top emission devices

For top emission devices - Examples 2 and 3, the anode electrode was formed from 100 nm silver on glass which is prepared by the same methods as described above for bottom emission devices.

The HIL, HTL, EML and organic semiconductor layer are deposed as described above for bottom emission devices.

Then the cathode is deposited. In example 2, a layer of 13 nm Ag is formed in high vacuum as described for bottom emission devices above. In example 3, a layer of 100 nm ITO is formed using a sputtering process.

60 nm biphenyl-4-yl(9,9-diphenyl-9H-fluoren-2-yl)-[4-(9-phenyl-9H-carbazol-3-yl)phenyl]-amine (CAS 1242056-42-3) is deposed directly on top of the cathode electrode layer.

The OLED stack is protected from ambient conditions by encapsulation of the device with a glass slide. Thereby, a cavity is formed, which includes a getter material for further protection.

Pn junction device as model for an OLED comprising at least two emission layers

The fabrication of OLEDs comprising at least two emission layers is time-consuming and expensive. Therefore, the effectiveness of the organic semiconductor layer of the present invention in a pn junction was tested without emission layers. In this arrangement, the organic semiconductor layer functions as n-type charge generation layer (CGL) and is arranged between the anode electrode and the cathode electrode and is in direct contact with the p-type CGL.

For pn junction devices - Examples 4 to 5 and comparative example 6, a 15Ω /cm 2 glass substrate (available from Corning Co.) with 100 nm Ag was cut to a size of 50 mm x 50 mm x 0.7 mm, ultrasonically cleaned with isopropyl alcohol for 5 minutes and then with pure water for 5 minutes, and cleaned again with UV ozone for 30 minutes, to prepare a first electrode.

Then, 97 wt.-% of Biphenyl-4-yl(9,9-diphenyl-9H-fluoren-2-yl)-[4-(9-phenyl-9H-carbazol-3-yl)phenyl]-amine (CAS 1242056-42-3) and 3 wt.-% of 2,2',2"-(cyclopropane-1,2,3-triylidene)tris(2-(p-cyanotetrafluorophenyl)acetonitrile) was vacuum deposited on the ITO electrode, to form a HIL having a thickness of 10 nm. Then 2,4-diphenyl-6-(3'-(triphenylen-2-yl)-[1,1'-biphenyl]-3-yl)-1,3,5-triazine (CAS 1638271-85-8) was vacuum deposited on the HIL, to form an electron blocking layer (EBL) having a thickness of 130 nm.

Then, the organic semiconductor layer is formed by deposing a matrix compound and metal dopant according to examples 4 and 5 and comparative example 6 by deposing the matrix compound from a first deposition source and rare earth metal dopant from a second deposition source directly on the EBL. The composition of the organic semiconductor layer can be seen in Table 2. In examples 4 and 5 the matrix compound is a compound of formula 1. The thickness of the organic semiconductor layer is 10 nm.

Then, the p-type CGL is formed by deposing the host and p-type dopant directly onto the organic semiconductor layer. The composition of the p-type CGL can be seen in Table 2. In comparative example 6, a layer of 10 nm formula (17) was deposited. In examples 4 and 5, 97 wt.-% of Biphenyl-4-yl(9,9-diphenyl-9H-fluoren-2-yl)-[4-(9-phenyl-9H-carbazol-3-yl)phenyl]-amine, referred to as HT-1, and 3 wt.-% of 2,2',2"-(cyclopropane-1,2,3-triylidene)tris(2-(p-cyanotetrafluorophenyl)acetonitrile), referred to as Dopant 1, was vacuum deposited to form a p-type CGL having a thickness of 10 nm.

Then, a layer of 30 nm Biphenyl-4-yl(9,9-diphenyl-9H-fluoren-2-yl)-[4-(9-phenyl-9H-carbazol-3-yl)phenyl]-amine is deposed directly on the p-type CGL to form a hole blocking layer (HBL).

Then, the cathode electrode layer is formed by evaporating aluminium at ultra-high vacuum of $10^{-7}$ bar and deposing the aluminium layer directly on the organic semiconductor layer. A thermal single co-evaporation of one or several metals is performed with a rate of 0, 1 to 10 nm/s (0.01 to 1 Å/s) in order to generate a homogeneous cathode electrode with a thickness of 5 to 1000 nm. The thickness of the cathode electrode layer is 100 nm.

The pn junction device is protected from ambient conditions by encapsulation of the device with a glass slide. Thereby, a cavity is formed, which includes a getter material for further protection.

To assess the performance of the inventive examples compared to the prior art, the current efficiency is measured under ambient conditions (20°C). Current voltage measurements are performed using a Keithley 2400 sourcemeter, and recorded in V. At 10 mA/cm$^2$ for bottom emission and 10 mA/cm$^2$ for top emission devices, a calibrated spectrometer CAS140 from Instrument Systems is used for measurement of CIE coordinates and brightness in Candela. Lifetime LT of bottom emission device is measured at ambient conditions (20°C) and 10 mA/cm$^2$, using a Keithley 2400 sourcemeter, and recorded in hours. Lifetime LT of top emission device is measured at ambient conditions (20°C) and 8 mA/cm$^2$. The brightness of the device is measured using a calibrated photo diode. The lifetime LT is defined as the time till the brightness of the device is reduced to 97 % of its initial value.

In bottom emission devices, the emission is predominately Lambertian and quantified in percent external quantum efficiency (EQE). To determine the efficiency EQE in % the light output of the device is measured using a calibrated photodiode at 10 mA/cm2.

In top emission devices, the emission is forward directed, non-Lambertian and also highly dependent on the mirco-cavity. Therefore, the efficiency EQE will be higher compared to bottom emission devices. To determine the efficiency EQE in % the light output of the device is measured using a calibrated photodiode at 10 mA/cm2.

The voltage rise over time is measured at a current density of 30 mA/cm$^2$ and 85 °C over 100 hours. The voltage rise is recorded in Volt (V).

In pn junction devices, the operating voltage is determined at 10 mA/cm$^2$ as described for OLEDs above.

Technical Effect of the invention

1. Organic semiconductor layer in direct contact with the cathode electrode (not in accordance with the invention)

In Table 1, operating voltage, external quantum efficiency and voltage rise over time are shown for OLEDs comprising a fluorescent blue emission layer, an organic semiconductor layer comprising a first matrix compound and a metal dopant and various cathode electrodes.

In comparative examples 1 to 3, ETM-1 is used as first matrix compound

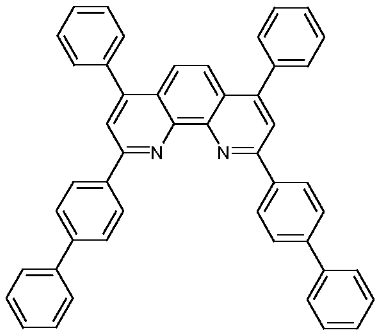

ETM-1.

ETM-1 comprises a single phenanthrolinyl group. Various metal dopants have been tested and the operating voltage is between 4.4 and 6.7 V and the external quantum efficiency is between 1.9 and 3.6 % EQE.

In comparative examples 4 and 5, MX1 is used as first matrix compound. MX 1 comprises two phenanthrolinyl groups. In comparative example 4, Li is used as metal dopant. The operating voltage is 3.3 V and the external quantum efficiency is 5.2 % EQE. The voltage rise over time at 85°C is 0.18 V. In comparative example 5, Mg is used as metal dopant. The operative voltage is 6 V and the external quantum efficiency is 4.3 % EQE. To check reproducibility of the metal doping concentration over several fabrication runs, the standard deviation for the actual concentration of metal dopant in the organic semiconductor layer and its impact on the operating voltage have been determined. In comparative example 4, the doping concentration varies by 0.06 mol.-% and the operating voltage varies by 0.09 V. This is a substantial variation in operating voltage which may result in a large number of devices not meeting the product specification.

In example 1, MX1 is used a first matrix compound and Yb as metal dopant. The operating voltage is very low at 3.8 V and the efficiency is further improved to 5.6 % EQE. Yb is significantly less hazardous to use than alkali metals and alkaline earth metals. Additionally, the voltage rise over time is significantly lower at 0.04 V compared to 0.18 V in comparative example 4. A further benefit of rare earth metal dopants is that a higher doping concentration can be used compared to Li. In comparative example 4, which is closest in operating voltage, 0.6 wt.-% Li is used. In example 1, 11.1 wt.-% Yb is used. The standard deviation for the actual Yb concentration in the organic semiconductor layer is 0.04 and the standard deviation for the operating voltage is 0.02. In summary, external quantum efficiency, voltage rise over time and standard deviation in operating voltage have been significantly improved.

In example 2, MX1 is used as first matrix compound and Yb as metal dopant. The anode electrode is formed from 100 nm Ag and the cathode electrode is formed from 13 nm Ag. As the cathode electrode is very thin, it is transparent to visible light emission. The efficiency is increased further to 7 % EQE.

In example 3, the same composition is used in the organic semiconductor layer as in example 2. The cathode electrode is formed from 100 nm ITO which is transparent to visible light emission. The efficiency is still very high at 6.6 % EQE and the operating voltage is low at 3.9 V.

In summary, a significant improvement in external quantum efficiency, reproducibility of metal doping concentration and voltage stability over time at elevated temperature has been achieved. Additionally, the operating voltage is still low while allowing safe handling of the rare earth metal dopants while loading the VTE source and reduced safety concerns during maintenance of the evaporation tool.

2. Organic semiconductor layer in direct contact with the p-type CGL

In Table 2, operating voltages are shown for pn junction devices comprising a p-type CGL and an organic semiconductor layer comprising a first matrix compound and a metal dopant and various cathode electrodes.

In comparative example 6, formula (17) is used as p-type CGL. The organic semiconductor layer comprises ETM-1 and Yb metal dopant. ETM-1 comprises a single phenanthrolinyl group. The operating voltage is 7.2 V.

In example 4, again formula (17) is used as p-type CGL. The organic semiconductor layer comprised MX1 and Yb metal dopant. MX1 comprises two phenanthrolinyl groups. The operating voltage is significantly improved to 4.9 V.

In example 5, HT-1 and Dopant 1 are co-deposited to form the p-type CGL. The organic semiconductor layer comprises MX1 and Yb metal dopant. The operating voltage is improved further to 4.8 V.

A lower operating voltage offers the benefit of lower power consumption and longer battery life in mobile devices.

The features disclosed in the foregoing description, in the claims and the accompanying drawings may, both separately or in any combination thereof be material for realizing the invention in diverse forms thereof.

Table 1: Device performance of organic light emitting diodes (not in accordance with the invention) comprising the organic semiconductor layer of the present invention in direct contact with the cathode electrode

| | Anode electrode | First matrix compound | Metal dopant | wt.-% metal dopant | mol.-% metal dopant | Cathode electrode | Thickness cathode electrode [nm] | Voltage at 10mA/cm$^2$ [V] | EQE at 10mA/cm$^2$ [%] | V rise at 30mA/cm$^2$ at 85°C [V] |
|---|---|---|---|---|---|---|---|---|---|---|
| Comparative example 1 | ITO | ETM-1 | Li | 0.6 | 34 | Al | 100 | 4.4 | 3.4 | - |
| Comparative example 2 | ITO | ETM-1 | Mg | 1.6 | 30 | Al | 100 | 6.7 | 1.9 | - |
| Comparative example 3 | ITO | ETM-1 | Yb | 10.5 | 30 | Al | 100 | 5.0 | 3.6 | - |
| Comparative example 4 | ITO | MX1 | Li | 0.6 | 33 | Al | 100 | 3.3 | 5.2 | 0.18 |
| Comparative example 5 | ITO | MX1 | Mg | 1.7 | 30 | Al | 100 | 6.0 | 4.3 | - |
| Example 1(not in accordance with the invention) | ITO | MX1 | Yb | 11.1 | 30 | Al | 100 | 3.8 | 5.6 | 0.04 |
| Example 2(not in accordance with the invention) | Ag | MX1 | Yb | 4.9 | 15 | Ag | 13 | 4.2 | 7.0 | - |
| Example 3(not in accordance with the invention) | Ag | MX1 | Yb | 4.9 | 15 | ITO | 100 | 3.9 | 6.6 | - |

Table 2: Device performance of pn junction devices comprising the organic semiconductor layer of the present invention in direct contact with the p-type charge generation layer (CGL)

| | p-type CGL | First matrix compound | Metal dopant | wt.-% metal dopant | mol.-% metal dopant | Voltage at 10mA/cm$^2$ [V] |
|---|---|---|---|---|---|---|
| Comparative example 6 | Formula (17) | ETM-1 | Yb | 9.5 | 28 | 7.2 |
| Example 4 | Formula (17) | MX1 | Yb | 11.4 | 30 | 4.9 |
| Example 5 | HT-1 : Dopant 1 | MX1 | Yb | 11.2 | 30 | 4.8 |

## Claims

**1.** Organic light emitting diode (100) comprising an anode electrode (120), a cathode electrode (190), at least one emission layer (150) and at least one organic semiconductor layer (170), wherein the at least one emission layer (150) and the at least one organic semiconductor layer (170) are arranged between the anode electrode (120) and the cathode electrode (190) and the at least one organic semiconductor layer (170) comprises a substantially metallic zero valent rare earth metal dopant selected from Sm, Eu, and Yb and a first matrix compound, the first matrix compound comprising two phenanthrolinyl groups, **characterized in that**

the organic light emitting diode comprises a first emission layer (150) and a second emission layer (151), wherein the organic semiconductor layer (170) is arranged between the first emission layer (150) and the second emission layer (151);
the organic light emitting diode (100) further comprises a p-type charge generation layer (135), wherein the organic semiconductor layer (170) is arranged between the first emission layer (150) and the p-type charge generation layer (135); and
the organic semiconductor layer (170) is in direct contact with the p-type charge generation layer (135).

**2.** Organic light emitting diode (100) according to claim 1, wherein the first matrix compound is a compound of Formula 1

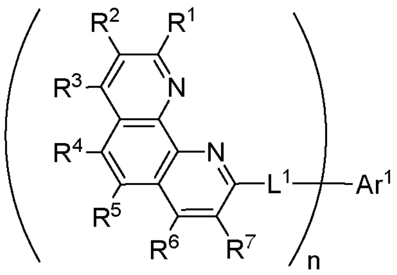

Formula 1

wherein $R^1$ to $R^7$ are each independently selected from the group consisting of hydrogen, substituted or unsubstituted $C_6$ to $C_{18}$ aryl group, substituted or unsubstituted pyridyl group, substituted or unsubstituted quinolyl group, substituted or unsubstituted $C_1$ to $C_{16}$ alkyl group, substituted or unsubstituted $C_1$ to $C_{16}$ alkoxy group, hydroxyl group or carboxyl group, and/or wherein adjacent groups of the respective $R^1$ to $R^7$ may be bonded to each other to form a ring;
$L^1$ is a single bond or selected from a group consisting of a $C_6$ to $C_{30}$ arylene group, a $C_5$ to $C_{30}$ heteroarylene group, a $C_1$ to $C_8$ alkylene group or a $C_1$ to $C_8$ alkoxyalkylene group;
$Ar^1$ is a substituted or unsubstituted $C_6$ to $C_{18}$ aryl group or a pyridyl group; and
n is 2, wherein each of the n phenanthrolinyl groups within the parentheses may be the same or different from each other.

**3.** Organic light emitting diode (100) according to any of the preceding claims, wherein the p-type charge generation layer (135) is composed of organic material doped with p-type dopant, wherein the p-type dopant is selected from a group consisting of tetrafluor-7,7,8,8-tetracyanoquinodimethane (F4-TCNQ), a derivative of tetracyanoquinodimethane, a radialene derivative, preferably, the p-type dopant is selected from radialene derivatives.

4. Organic light emitting diode (100) according to any of the preceding claims, wherein the p-type charge generation layer (135) comprises, preferably consists of, a radialene dopant and a host.

5. Organic light emitting diode (100) according to any of the preceding claims, wherein the p-type charge generation layer (135) comprises an aryl amine-based compound including a compounds of the following Chemical Formula 18:

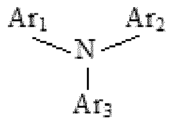

(18),

wherein
$Ar_1$, $Ar_2$ and $Ar_3$ are each independently hydrogen or a hydrocarbon group, preferably at least one of $Ar_1$, $Ar_2$ and $Ar_3$ includes aromatic hydrocarbon substituents, and each substituent is the same, or different, wherein if $Ar_1$, $Ar_2$ and $Ar_3$ are not aromatic hydrocarbons, $Ar_1$, $Ar_2$ and $Ar_3$ are selected from hydrogen; a straight-chain aliphatic hydrocarbon, a branched-chain aliphatic hydrocarbon, a cyclic aliphatic hydrocarbon; or a heterocyclic group including N, O, S or Se.

6. Organic light emitting diode (100) according to any of the preceding claims, further comprising an electron transport layer (160) which is arranged between the at least one emission layer (150) and the at least one organic semiconductor layer (170).

7. Organic light emitting diode (100) according to any of the preceding claims, wherein the p-type charge generation layer (135) is arranged between the organic semiconductor layer (170) and the cathode electrode (190).

8. Organic light emitting diode (100) according to any of the preceding claims, wherein the cathode electrode (190) is transparent to visible light emission.

9. Organic light emitting diode (100) according to any of the preceding claims, wherein the cathode electrode (190) comprises a first cathode electrode layer (191) and a second cathode electrode layer (192).

10. Organic light emitting diode (100) according to any of the preceding claims 2 to 9, wherein $L^1$ is a single bond.

11. Organic light emitting diode (100) according to any of the preceding claims 2 to 9, wherein $Ar^1$ is phenylene.

12. Organic light emitting diode (100) according to any of the preceding claims 2 to 9, wherein $R^1$ to $R^7$ are independently selected from the group consisting of hydrogen, $C_1$ to $C_4$ alkyl group, $C_1$ to $C_4$ alkoxy group, $C_6$ to $C_{12}$ aryl group and $C_5$ to $C_{12}$ heteroaryl group, preferably from hydrogen, $C_1$ to $C_4$ alkyl group and phenyl.

13. A method of manufacturing an organic light emitting diode (100) according to any of the preceding claims, comprising the steps of sequentially forming an anode electrode (120), at least one emission layer (150), at least one organic semiconductor layer (170), and a cathode electrode (190) on a substrate (110), and forming the at least one organic semiconductor layer (170) by co-depositing a substantially metallic zero valent rare earth metal dopant selected from Sm, Eu and Yb together with a first matrix compound comprising at least two phenanthrolinyl groups.

## Patentansprüche

1. Organische Leuchtdiode (100), umfassend eine Anodenelektrode (120), eine Kathodenelektrode (190), mindestens eine Emissionsschicht (150) und mindestens eine organische Halbleiterschicht (170), wobei die mindestens eine Emissionsschicht (150) und die mindestens eine organische Halbleiterschicht (170) zwischen der Anodenelektrode (120) und der Kathodenelektrode (190) angeordnet sind und die mindestens eine organische Halbleiterschicht (170) einen im Wesentlichen metallischen nullwertigen Seltenerdmetall-Dotierstoff, ausgewählt aus Sm, Eu und Yb, und eine erste Matrixverbindung umfasst, wobei die erste Matrixverbindung zwei Phenanthrolinylgruppen umfasst, **dadurch gekennzeichnet, dass**

die organische Leuchtdiode eine erste Emissionsschicht (150) und eine zweite Emissionsschicht (151) umfasst, wobei die organische Halbleiterschicht (170) zwischen der ersten Emissionsschicht (150) und der zweiten

Emissionsschicht (151) angeordnet ist; die organische Leuchtdiode (100) ferner eine p-Typ-Ladungserzeugungsschicht (135) umfasst, wobei die organische Halbleiterschicht (170) zwischen der ersten Emissionsschicht (150) und der p-Typ-Ladungserzeugungsschicht (135) angeordnet ist; und
die organische Halbleiterschicht (170) in direktem Kontakt mit der p-Typ-Ladungserzeugungsschicht (135) steht.

**2.** Organische Leuchtdiode (100) nach Anspruch 1, wobei die erste Matrixverbindung eine Verbindung der Formel 1 ist

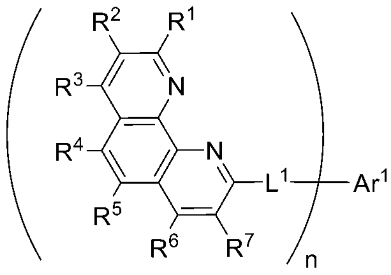

Formula 1

wobei $R^1$ bis $R^7$ jeweils unabhängig ausgewählt sind aus der Gruppe, bestehend aus Wasserstoff, einer substituierten oder unsubstituierten $C_6$ - bis $C_{18}$ -Arylgruppe, einer substituierten oder unsubstituierten Pyridylgruppe, einer substituierten oder unsubstituierten Chinolylgruppe, einer substituierten oder unsubstituierten $C_1$ - bis $C_{16}$ -Alkylgruppe, einer substituierten oder unsubstituierten $C_1$ - bis $C_{16}$ -Alkoxygruppe, einer Hydroxylgruppe oder einer Carboxylgruppe, und/oder wobei benachbarte Gruppen der jeweiligen $R^1$ bis $R^7$ aneinander gebunden sein können, um einen Ring zu bilden;
$L^1$ eine Einfachbindung ist oder ausgewählt ist aus einer Gruppe, bestehend aus einer $C_6$ - bis $C_{30}$ -Arylengruppe, einer $C_5$ - bis $C_{30}$ -Heteroarylengruppe, einer $C_1$ - bis $C_8$ - Alkylengruppe oder einer $C_1$ - bis $C_8$ -Alkoxyalkylengruppe;
$Ar^1$ eine substituierte oder unsubstituierte $C_6$ - bis $C_{18}$ -Arylgruppe oder eine Pyridylgruppe ist; und
n 2 ist, wobei jede der n Phenanthrolinylgruppen in den Klammern gleich oder verschieden voneinander sein kann.

**3.** Organische Leuchtdiode (100) nach einem der vorhergehenden Ansprüche, wobei die p-Typ-Ladungserzeugungsschicht (135) aus organischem Material besteht, das mit einem p-Typ-Dotierstoff dotiert ist, wobei der p-Typ-Dotierstoff ausgewählt ist aus einer Gruppe, bestehend aus Tetrafluor-7,7,8,8-tetracyanochinodimethan (F4-TCNQ), einem Derivat von Tetracyanochinodimethan, einem Radialenderivat, wobei der p-Typ-Dotierstoff vorzugsweise aus Radialenderivaten ausgewählt ist.

**4.** Organische Leuchtdiode (100) nach einem der vorhergehenden Ansprüche, wobei die p-Typ-Ladungserzeugungsschicht (135) einen Radialendotierstoff und einen Wirt umfasst, vorzugsweise daraus besteht.

**5.** Organische Leuchtdiode (100) nach einem der vorhergehenden Ansprüche, wobei die p-Typ-Ladungserzeugungsschicht (135) eine Verbindung auf Arylaminbasis umfasst, die eine Verbindung der folgenden chemischen Formel 18 einschließt:

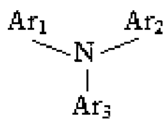

(18),

wobei
$Ar_1$, $Ar_2$ und $Ar_3$ jeweils unabhängig Wasserstoff oder eine Kohlenwasserstoffgruppe sind, wobei vorzugsweise mindestens eines von $Ar_1$, $Ar_2$ und $Ar_3$ aromatische Kohlenwasserstoffsubstituenten einschließt und jeder Substituent gleich oder verschieden ist, wobei, wenn $Ar_1$, $Ar_2$ und $Ar_3$ keine aromatischen Kohlenwasserstoffe sind, $Ar_1$, $Ar_2$ und $Ar_3$ ausgewählt sind aus Wasserstoff; einem geradkettigen aliphatischen Kohlenwasserstoff, einem verzweigtkettigen aliphatischen Kohlenwasserstoff, einem cyclischen aliphatischen Kohlenwasserstoff; oder einer heterocyclischen Gruppe, die N, O, S oder Se einschließt.

**6.** Organische Leuchtdiode (100) nach einem der vorhergehenden Ansprüche, ferner umfassend eine Elektronentransportschicht (160), die zwischen der mindestens einen Emissionsschicht (150) und der mindestens einen organischen Halbleiterschicht (170) angeordnet ist.

**7.** Organische Leuchtdiode (100) nach einem der vorhergehenden Ansprüche, wobei die p-Typ-Ladungserzeugungsschicht (135) zwischen der organischen Halbleiterschicht (170) und der Kathodenelektrode (190) angeordnet ist.

**8.** Organische Leuchtdiode (100) nach einem der vorhergehenden Ansprüche, wobei die Kathodenelektrode (190) für sichtbares Licht transparent ist.

**9.** Organische Leuchtdiode (100) nach einem der vorhergehenden Ansprüche, wobei die Kathodenelektrode (190) eine erste Kathodenelektrodenschicht (191) und eine zweite Kathodenelektrodenschicht (192) umfasst.

**10.** Organische Leuchtdiode (100) nach einem der vorhergehenden Ansprüche 2 bis 9, wobei $L^1$ eine Einfachbindung ist.

**11.** Organische Leuchtdiode (100) nach einem der vorhergehenden Ansprüche 2 bis 9, wobei $Ar^1$ Phenylen ist.

**12.** Organische Leuchtdiode (100) nach einem der vorhergehenden Ansprüche 2 bis 9, wobei $R^1$ bis $R^7$ unabhängig ausgewählt sind aus der Gruppe, bestehend aus Wasserstoff, einer $C_1$ - bis $C_4$ -Alkylgruppe, einer $C_1$ - bis $C_4$ -Alkoxygruppe, einer $C_6$ - bis $C_{12}$ -Arylgruppe und einer $C_5$ - bis $C_{12}$ -Heteroarylgruppe, vorzugsweise aus Wasserstoff, einer $C_1$ - bis $C_4$ -Alkylgruppe und Phenyl.

**13.** Verfahren zum Herstellen einer organischen Leuchtdiode (100) nach einem der vorhergehenden Ansprüche, umfassend die Schritte des aufeinanderfolgenden Bildens einer Anodenelektrode (120), mindestens einer Emissionsschicht (150), mindestens einer organischen Halbleiterschicht (170) und einer Kathodenelektrode (190) auf einem Substrat (110) und Bilden der mindestens einen organischen Halbleiterschicht (170) durch gemeinsames Abscheiden eines im Wesentlichen metallischen nullwertigen Seltenerdmetall-Dotierstoffs, ausgewählt aus Sm, Eu und Yb, zusammen mit einer ersten Matrixverbindung, die mindestens zwei Phenanthrolinylgruppen umfasst.

## Revendications

**1.** Diode électroluminescente organique (100) comprenant une électrode d'anode (120), une électrode de cathode (190), au moins une couche d'émission (150) et au moins une couche semi-conductrice organique (170), dans laquelle la au moins une couche d'émission (150) et la au moins une couche semi-conductrice organique (170) sont disposées entre l'électrode d'anode (120) et l'électrode de cathode (190), et la au moins une couche semi-conductrice organique (170) comprend un dopant métallique à valence zéro de métal des terres rares choisi parmi Sm, Eu et Yb, et un premier composé matriciel, le premier composé matriciel comprenant deux groupes phénanthrolinyle, **caractérisée en ce que**

la diode électroluminescente organique comprend une première couche d'émission (150) et une seconde couche d'émission (151), dans laquelle la couche semi-conductrice organique (170) est disposée entre la première couche d'émission (150) et la seconde couche d'émission (151) ;
la diode électroluminescente organique (100) comprend en outre une couche de génération de charge de type p (135), dans laquelle la couche semi-conductrice organique (170) est disposée entre la première couche d'émission (150) et la couche de génération de charge de type p (135) ; et
la couche semi-conductrice organique (170) est en contact direct avec la couche de génération de charge de type p (135).

**2.** Diode électroluminescente organique (100) selon la revendication 1, dans laquelle le premier composé matriciel est un composé de formule 1

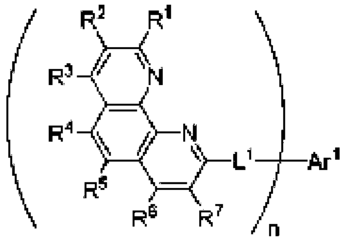

Formule 1

dans laquelle $R^1$ à $R^7$ sont chacun indépendamment choisis dans le groupe constitué par l'hydrogène, un groupe aryle en $C_6$ à $C_{18}$ substitué ou non substitué, un groupe pyridyle substitué ou non substitué, un groupe quinolyle substitué ou non substitué, un groupe alkyle en $C_1$ à $C_{16}$ substitué ou non substitué, un groupe alcoxy en $C_1$ à $C_{16}$ substitué ou non substitué, un groupe hydroxyle ou un groupe carboxyle, et/ou dans laquelle les groupes adjacents de $R_1$ à $R^7$ respectifs peuvent être liés les uns aux autres pour former un cycle ;
$L^1$ représente une liaison simple ou est choisi dans le groupe constitué par un groupe arylène en $C_6$ à $C_{30}$, un groupe hétéroarylène en Cs à $C_{30}$, un groupe alkylène en $C_1$ à $C_8$ ou un groupe alcoxyalkylène en $C_1$ à $C_8$ ;
$Ar^1$ représente un groupe aryle en $C_6$ à $C_{18}$ substitué ou non substitué ou un groupe pyridyle ; et
n vaut 2, chacun des n groupes phénanthrolinyle entre parenthèses pouvant être identique ou différent les uns des autres.

**3.** Diode électroluminescente organique (100) selon l'une quelconque des revendications précédentes, dans laquelle la couche de génération de charge de type p (135) est composée d'un matériau organique dopé avec un dopant de type p, dans laquelle le dopant de type p est choisi parmi un groupe comprenant le tétrafluoro-7,7,8,8-tétracyanoquinodiméthane (F4-TCNQ), un dérivé du tétracyanoquinodiméthane, un dérivé du radialène, de préférence, le dopant de type p est choisi parmi les dérivés du radialène.

**4.** Diode électroluminescente organique (100) selon l'une quelconque des revendications précédentes, dans laquelle la couche de génération de charge de type p (135) comprend, de préférence est constituée d'un dopant radialène et d'un hôte.

**5.** Diode électroluminescente organique (100) selon l'une quelconque des revendications précédentes, dans laquelle la couche de génération de charge de type p (135) comprend un composé à base d'arylamine comprenant des composés de formule chimique 18 suivante :

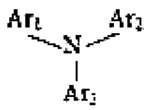

(18),

dans laquelle
$Ar_1$, $Ar_2$ et $Ar_3$ représentent chacun indépendamment un atome d'hydrogène ou un groupe hydrocarboné, de préférence au moins l'un parmi $Ar_1$, $Ar_2$ et $Ar_3$ comprend des substituants hydrocarbonés aromatiques, et chaque substituant est identique ou différent, dans lequel si $Ar_1$, $Ar_2$ et $Ar_3$ ne sont pas des hydrocarbures aromatiques, $Ar_1$, $Ar_2$ et $Ar_3$ sont choisis parmi l'hydrogène ; un hydrocarbure aliphatique à chaîne linéaire, un hydrocarbure aliphatique à chaîne ramifiée, un hydrocarbure aliphatique cyclique ; ou un groupe hétérocyclique comprenant N, O, S ou Se.

**6.** Diode électroluminescente organique (100) selon l'une quelconque des revendications précédentes, comprenant en outre une couche de transport d'électrons (160) qui est disposée entre la au moins une couche d'émission (150) et la au moins une couche semi-conductrice organique (170).

**7.** Diode électroluminescente organique (100) selon l'une quelconque des revendications précédentes, dans laquelle la couche de génération de charge de type p (135) est disposée entre la couche semi-conductrice organique (170) et l'électrode de cathode (190).

**8.** Diode électroluminescente organique (100) selon l'une quelconque des revendications précédentes, dans laquelle l'électrode de cathode (190) est transparente à l'émission de lumière visible.

**9.** Diode électroluminescente organique (100) selon l'une quelconque des revendications précédentes, dans laquelle l'électrode de cathode (190) comprend une première couche d'électrode de cathode (191) et une seconde couche d'électrode de cathode (192).

**10.** Diode électroluminescente organique (100) selon l'une quelconque des revendications 2 à 9 précédentes, dans laquelle $L^1$ représente une liaison simple.

**11.** Diode électroluminescente organique (100) selon l'une quelconque des revendications 2 à 9 précédentes, dans laquelle $Ar^1$ représente un groupe phénylène.

**12.** Diode électroluminescente organique (100) selon l'une quelconque des revendications 2 à 9 précédentes, dans laquelle $R^1$ à $R^7$ sont choisis indépendamment dans le groupe constitué par l'hydrogène, un groupe alkyle en $C_1$ à $C_4$, un groupe alcoxy en $C_1$ à $C_4$, un groupe aryle en $C_6$ à $C_{12}$ et un groupe hétéroaryle en $C_5$ à $C_{12}$, de préférence parmi l'hydrogène, un groupe alkyle en $C_1$ à $C_4$ et un groupe phényle.

**13.** Procédé de fabrication d'une diode électroluminescente organique (100) selon l'une quelconque des revendications précédentes, comprenant les étapes consistant à former séquentiellement une électrode d'anode (120), au moins une couche d'émission (150), au moins une couche semi-conductrice organique (170) et une électrode de cathode (190) sur un substrat (110), et la formation de la au moins une couche semi-conductrice organique (170) par co-dépôt d'un dopant métallique à valence zéro choisi parmi Sm, Eu et Yb, conjointement avec un premier composé matriciel comprenant au moins deux groupes phénanthrolinyle.

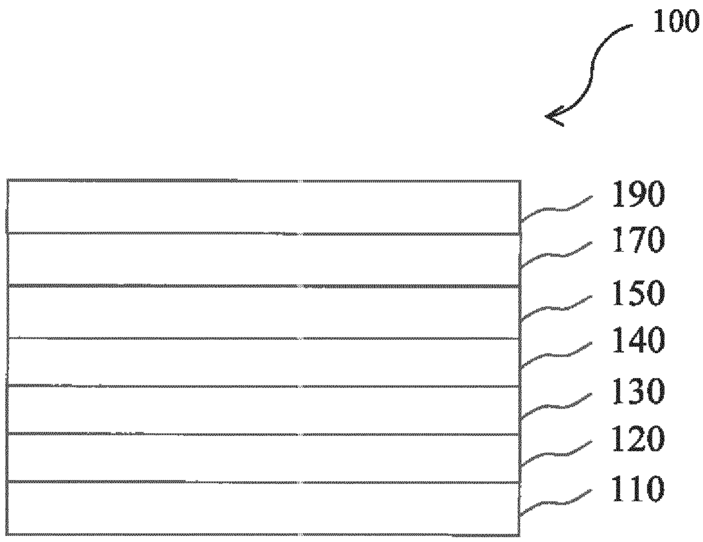
100
190
170
150
140
130
120
110

Fig.1

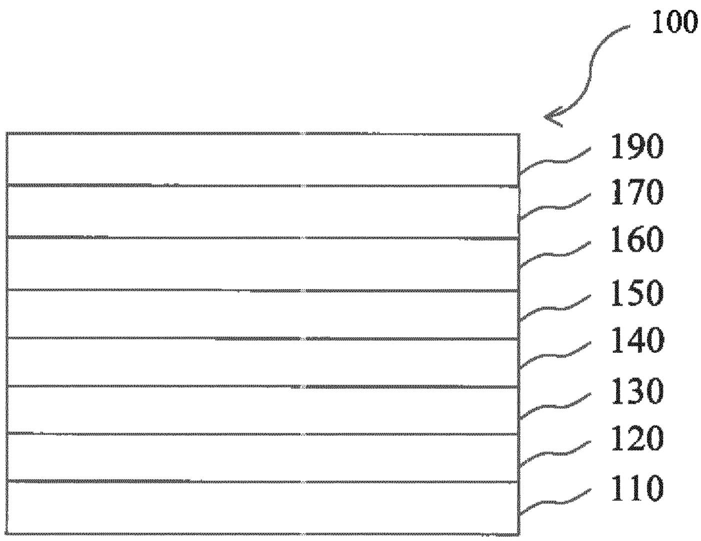
100
190
170
160
150
140
130
120
110

Fig.2

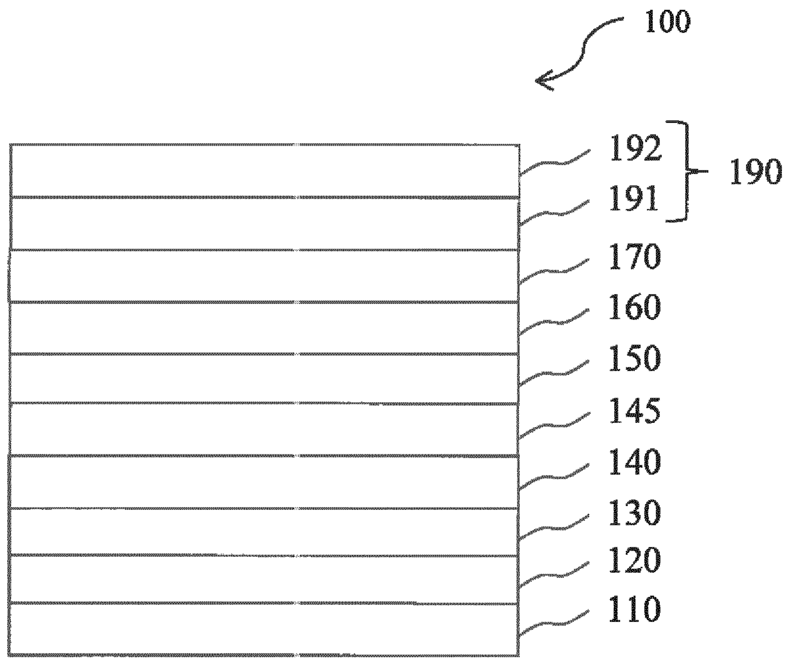


Fig.3

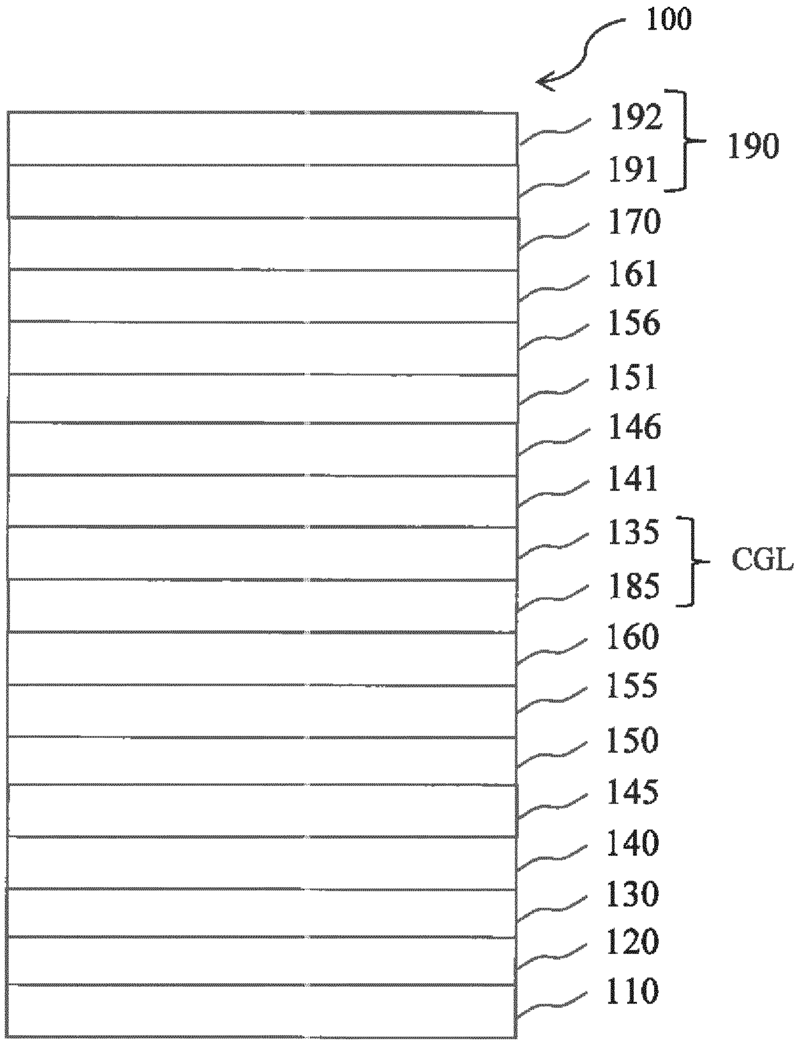
100
192
191
190
170
161
156
151
146
141
135
185
CGL
160
155
150
145
140
130
120
110

Fig.4

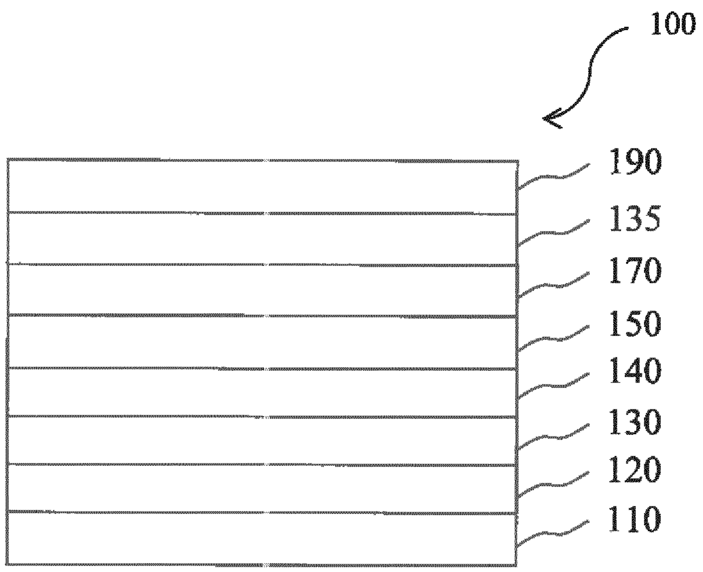


Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2833429 A1
- JP 2008243932 A
- JP 2008177459 A
- EP 2833700 A1
- EP 2722908 A1
- EP 15201418
- EP 2395571 A
- EP 2312663 A
- WO 2015083948 A
- US 2011284832 A
- WO 2014171541 A
- WO 2015008866 A
- WO 2015105313 A
- JP 2015074649 A
- JP 2015126140 A
- KR 20150088712
- WO 2010020352 A
- WO 20150105316 A
- JP 10189247 A
- US 2007202354 A
- US 20050025993 A
- JP 2005032686 B
- US 2003168970 A
- WO 2013149958 A
- US 2015207079 A
- KR 2011085784
- EP 2395571 A1
- WO 2013079217 A1
- EP 13187905 A
- EP 13199361 A
- JP 2002063989 A
- EP 15195877
- US 6878469 B
- WO 2010134352 A
- US 20090108746 A
- US 20090166670 A
- JP 2004281390 B
- US 20120280613 A
- JP 2004031004 B
- JP 2003007467 B
- WO 2014163173 A
- US 2015280160 A
- EP 2504871 A
- EP 1786050 A1
- CN 102372708
- KR 2012102374
- KR 2014076522
- EP 1970371 A
- CN 104650032
- WO 2016001283 A1
- US 2012098012 A
- JP 2002352961 B


### Non-patent literature cited in the description


- **YASUHIKO SHIROTA** ; **HIROSHI KAGEYAMA**. *Chem. Rev.*, 2007, vol. 107, 953-1010
- **ADACHI, C.** ; **TOKITO, S.** ; **TSUTSUI, T.** ; **SAITO, S.** *Japanese Journal of Applied Physics, Part 2: Letters*, 1988, vol. 27 (2), L269-L271
- **SHIROTA** ; **KAGEYAMA**. *Chem. Rev.*, 2007, vol. 107, 953-1010
- *CHEMICAL ABSTRACTS*, 1242056-42-3
- *CHEMICAL ABSTRACTS*, 1638271-85-8